(19) Europäisches Patentamt
European Patent Office
Office européen des brevets

(11) Veröffentlichungsnummer: **0 403 883 A1**

(12) # EUROPÄISCHE PATENTANMELDUNG

(21) Anmeldenummer: **90110761.5**

(51) Int. Cl.$^5$: **A01N 47/44**

(22) Anmeldetag: **07.06.90**

(30) Priorität: **20.06.89 DE 3920086**

(43) Veröffentlichungstag der Anmeldung: **27.12.90 Patentblatt 90/52**

(84) Benannte Vertragsstaaten: **BE CH DE FR GB IT LI NL**

(71) Anmelder: **BAYER AG**
**D-5090 Leverkusen 1 Bayerwerk(DE)**

(72) Erfinder: **Gesing, Ernst Rudolf, Dr.**
**Trillser Graben 4**
**D-4006 Erkrath-Hochdahl(DE)**
Erfinder: **Fest, Christa, Dr.**
**Im Johannistal 20**
**D-5600 Wuppertal 1(DE)**
Erfinder: **Kirsten, Rolf, Dr.**
**Carl-Langhans-Strasse 27**
**D-4019 Monheim(DE)**
Erfinder: **Kluth, Joachim, Dr.**
**Tannenweg 9**
**D-4018 Langenfeld 9(DE)**
Erfinder: **Müller, Klaus-Helmut, Dr.**
**Bockhackstrasse 55**
**D-4000 Düsseldorf 13(DE)**
Erfinder: **Pfister, Theodor, Dr.**
**Lichtenberger Strasse 30**
**D-4019 Monheim(DE)**
Erfinder: **Riebel, Hans-Jochem, Dr.**
**In der Beek 92**
**D-5600 Wuppertal 1(DE)**
Erfinder: **Santel, Hans-Joachim, Dr.**
**Grünstrasse 9a**
**D-5090 Leverkusen 1(DE)**
Erfinder: **Lürssen, Klaus, Dr.**
**August-Kierspel-Strasse 145**
**D-5060 Bergisch Gladbach 2(DE)**
Erfinder: **Schmidt, Robert R., Dr.**
**Im Waldwinkel 110**
**D-5060 Bergisch Gladbach 2(DE)**

(54) **Herbizide Mittel auf Basis von sulfonylierten Aminoguanidinen.**

(57) Die Erfindung betrifft die Verwendung von sulfonylierten Aminoguanidinen als Herbizide.

Die sulfonylierten Aminoguanidine können durch die folgenden tautomeren Strukturformeln gekennzeichnet werden:

EP 0 403 883 A1

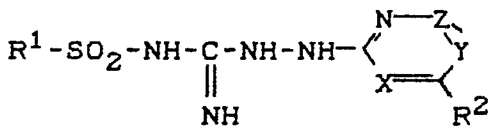

(I),

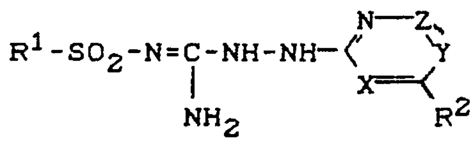

(IA) bzw.

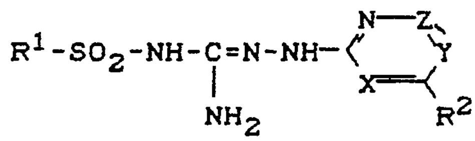

(IB),

worin

$R^1$ für einen gegebenenfalls substituierten Rest aus der Reihe Aryl, Aralkyl und Heteroaryl steht,

$R^2$ für Wasserstoff, Halogen, Hydroxy, Amino oder einen gegebenenfalls substituierten Rest aus der Reihe Alkyl, Cycloalkyl, Alkoxy, Alkylthio, Alkylamino und Dialkylamino steht,

X für Stickstoff oder die Gruppierung CH steht,

Y für Stickstoff oder die Gruppierung C-$R^3$ steht, worin

$R^3$ für Wasserstoff, Halogen, Cyano, Alkyl, Formyl, Alkylcarbonyl oder Alkoxycarbonyl steht, und

Z für Stickstoff oder die Gruppierung C-$R^4$ steht, worin

$R^4$ für Wasserstoff, Halogen, Hydroxy, Amino oder einen gegebenenfalls substituierten Rest aus der Reihe Alkyl, Cycloalkyl, Alkoxy, Alkylthio, Alkylamino und Dialkylamino steht.

## Herbizide Mittel auf Basis von sulfonylierten Aminoguanidinen

Die vorliegende Erfindung betrifft die Verwendung von sulfonylierten Aminoguanidinen als Herbizide.

Verschiedene Guanidin-Derivate, wie z.B. N′-(4,6-Dimethyl-pyrimidin-2-yl)-N″-(2-chlor-phenylsulfonyl)-guanidin, sind als potentielle Herbizide bekannt geworden, haben jedoch bisher als Mittel zur Unkrautbekämpfung keine Bedeutung erlangt (vgl. EP-A 121082, Bsp. 90).

Weiter sind sulfonylierte Aminoguanidine als Zwischenprodukte für Herbizide Gegenstand einer vorgängigen, jedoch nicht vorveröffentlichten Patentanmeldung der Anmelderin (vgl. DE-P 38 25 867.6 vom 29.07.1988).

Es wurde nun gefunden, daß die sulfonylierten Aminoguanidine der allgemeinen Formel (I)

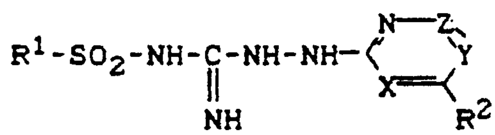

(I)

in welcher

$R^1$ für einen gegebenenfalls substituierten Rest aus der Reihe Aryl, Aralkyl und Heteroaryl steht,

$R^2$ für Wasserstoff, Halogen, Hydroxy, Amino oder einen gegebenenfalls substituierten Rest aus der Reihe Alkyl, Cycloalkyl, Alkoxy, Alkylthio, Alkylamino und Dialkylmino steht,

X für Stickstoff oder die Gruppierung CH steht,

Y für Stickstoff oder die Gruppierung C-$R^3$ steht, worin

$R^3$ für Wasserstoff, Halogen, Cyano, Alkyl, Formyl, Alkylcarbonyl oder Alkoxycarbonyl steht, und

Z für Stickstoff oder die Gruppierung C-$R^4$ steht, worin

$R^4$ für Wasserstoff, Halogen, Hydroxy, Amino oder einen gegebenenfalls substituierten Rest aus der Reihe Alkyl, Cycloalkyl, Alkoxy, Alkylthio, Alkylamino und Dialkylmino steht,

sehr gut zur Bekämpfung von Unkräutern geeignet sind.

Durch die allgemeine Formel (I) wird nur einer von drei möglichen tautomeren Strukturtypen wiedergegeben; die beiden anderen sind nachstehend skizziert:

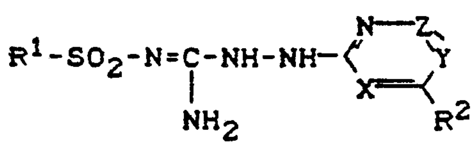

(IA)

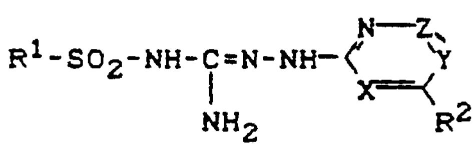

(IB)

Soweit in der Beschreibung der vorliegenden Erfindung von den Verbindungen der Formel (I) die Rede ist, sind darin jeweils die möglichen Tautomeren der oben angegebenen Formeln (I), (IA) und (IB) sowie die möglichen Gemische dieser Tautomeren zu verstehen.

Das Mischungsverhältnis hängt jeweils von aggregations-bestimmenden Faktoren, wie beispielsweise Temperatur, Lösungsmittel und Konzentration ab. Die vorliegende Erfindung betrifft also die Verwendung der Verbindungen der Formeln (I), (IA) und (IB) als Herbizide.

Überraschenderweise zeigen die erfindungsgemäß zu verwendenden sulfonylierten Aminoguanidine der allgemeinen Formel (I) ein wesentlich bessere Wirkung als die aus dem Stand der Technik bekannten Guanidin-Derivate, wie z.B. N′-(4,6-Dimethyl-pyrimidin-2-yl)-N″-(2-chlor-phenylsulfonyl)-guanidin (vgl. EP-A 121 082).

Die erfindungsgemäß zu verwendenden sulfonylierten Aminoguanidine sind durch die Formel (I) allgemein definiert. In dieser Formel steht vorzugsweise

$R^1$ für den Rest

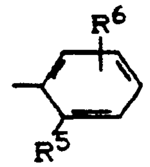

worin

$R^5$ und $R^6$ gleich oder verschieden sind und für Wasserstoff, Fluor, Chlor, Brom, Iod, Cyano, Nitro, $C_1$-$C_6$-Alkyl (welches gegebenenfalls durch Fluor, Chlor, Brom, Cyano, Carboxy, $C_1$-$C_4$-Alkoxycarbonyl, $C_1$-$C_4$-Alkylamino-carbonyl, Di-($C_1$-$C_4$-alkyl)amino-carbonyl, Hydroxy, $C_3$-$C_4$-Alkoxy, Formyloxy, $C_1$-$C_4$-Alkyl-carbonyloxy, $C_1$-$C_4$-Alkoxy-carbonyloxy, $C_1$-$C_4$-Alkylamino-carbonyloxy, $C_1$-$C_4$-Alkylthio, $C_1$-$C_4$-Alkylsulfinyl, $C_1$-$C_4$-Alkylsulfonyl, Di-($C_1$-$C_4$-alkyl)aminosulfonyl, $C_3$-$C_6$-Cycloalkyl oder Phenyl substituiert ist), für $C_2$-$C_6$-Alkenyl (welches gegebenenfalls durch Fluor, Chlor, Brom, Cyano, $C_3$-$C_4$-Alkoxy-carbonyl, Carboxy oder Phenyl substituiert ist), für $C_2$-$C_6$-Alkinyl (welches gegebenenfalls durch Fluor, Chlor, Brom, Cyano, $C_1$-$C_4$-Alkoxy-carbonyl; Carboxy oder Phenyl substituiert ist), für $C_1$-$C_4$-Alkoxy (welches gegebenenfalls durch Fluor, Chlor, Brom, Cyano, Carboxy, $C_1$-$C_4$-Alkoxy-carbonyl, $C_1$-$C_4$-Alkoxy, $C_1$-$C_4$-Alkylthio, $C_1$-$C_4$-Alkylsulfinyl oder $C_1$-$C_4$-Alkylsulfonyl substituiert ist), für $C_1$-$C_4$-Alkylthio (welches gegebenenfalls durch Fluor, Chlor, Brom, Cyano, Carboxy $C_1$-$C_4$-Alkoxy-carbonyl, $C_1$-$C_4$-Alkylthio, $C_1$-$C_4$-Alkylsulfinyl oder $C_1$-$C_4$-Alkylsulfonyl substituiert ist), für $C_3$-$C_6$-Alkenyloxy (welches gegebenenfalls durch Fluor, Chlor, Brom, Cyano oder $C_1$-$C_4$-Cyano oder $C_1$-$C_4$-Alkoxy-carbonyl substituiert ist), für $C_2$-$C_6$-Alkenylthio (welches gegebenenfalls durch Fluor, Chlor, Brom, Cyano, Nitro, $C_1$-$C_3$-Alkylthio oder $C_1$-$C_4$-Alkoxycarbonyl substituiert ist), $C_3$-$C_6$-Alkinyloxy, $C_3$-$C_6$-Alkinylthio oder für den Rest $-S(O)_p-R^7$ stehen, wobei

p für die Zahlen 1 oder 2 steht und

$R^7$ für $C_1$-$C_4$-Alkyl (welches gegebenenfalls durch Fluor, Chlor, Brom, Cyano oder $C_1$-$C_4$-Alkoxy-carbonyl substituiert ist), $C_3$-$C_6$-Alkenyl, $C_3$-$C_6$-Alkinyl, $C_1$-$C_4$-Alkoxy, $C_1$-$C_4$-Alkoxy-$C_1$-$C_4$-alkylamino, $C_1$-$C_4$-Alkylamino, Di-($C_1$-$C_4$-alkyl)-amino oder für den Rest $-NHOR^8$ steht, wobei

$R^8$ für $C_1$-$C_6$-Alkyl (welches gegebenenfalls durch Fluor, Chlor, Cyano, $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Alkyl-carbonyl, $C_1C_4$-Alkoxy-carbonyl, $C_1$-$C_4$-Alkylamino- carbonyl oder Di-($C_1$-$C_4$-alkyl)-amino-carbonyl substituiert ist), für $C_3$-$C_6$-Alkenyl (welches gegebenenfalls durch Fluor, Chlor oder Brom substituiert ist), $C_3$-$C_6$-Alkinyl, $C_3$-$C_6$-Cycloalkyl, $C_3$-$C_6$-Cycloalkyl-$C_1$-$C_2$-alkyl, Phenyl-$C_1$-$C_2$-alkyl (welches gegebenenfalls durch Fluor, Chlor, Nitro, Cyano, $C_1$-$C_4$-Alkyl, $C_1$-$C_4$-Alkoxy oder $C_1$-$C_4$-Alkoxy-carbonyl substituiert ist), für Benzhydryl oder für Phenyl (welches gegebenenfalls durch Fluor, Chlor, Nitro, Cyano, $C_1$-$C_4$-Alkyl, Trifluormethyl, $C_1$-$C_4$-Alkoxy, $C_1$-$C_2$-Fluoralkoxy, $C_1$-$C_4$-Alkylthio, Trifluormethylthio oder $C_1$-$C_4$-Alkoxy-carbonyl substituiert ist) steht,

$R^5$ und $R^6$ weiterhin für Phenyl oder Phenoxy, für $C_1$-$C_4$-Alkylcarbonylamino, $C_1$-$C_4$-Alkoxy-carbonylamino, $C_1$-$C_4$-Alkylamino-carbonyl-amino, Di-($C_1$-$C_4$-alkyl)-amino-carbonylamino, oder für den Rest $-CO-R^9$ stehen, wobei

$R^9$ für $C_1$-$C_6$-Alkyl, $C_1$-$C_6$-Alkoxy (welches gegebenenfalls durch Fluor und/oder Chlor substituiert ist), $C_3$-$C_6$-Cycloalkoxy, $C_3$-$C_6$-Alkenyloxy, $C_1$-$C_4$-Alkylthio, Amino, $C_1$-$C_4$-Alkylamino $C_1C_4$-Alkoxyamino, $C_1$-$C_4$-Alkoxy-$C_1$-$C_4$-alkyl-amino oder Di-($C_1$-$C_4$-alkyl)-amino steht;

$R^5$ und $R^6$ weiterhin für $C_1$-$C_4$-Alkylsulfonyloxy, Di-($C_1$-$C_4$-alkyl)-aminosulfonylamino oder für den Rest $-CH=N-R^{10}$ stehen, wobei

$R^{10}$ für gegebenenfalls durch Fluor, Chlor, Cyano, Carboxy, $C_1$-$C_4$-Alkoxy, $C_1$-$C_4$-Alkylthio, $C_1$-$C_4$-Alkylsulfinyl oder $C_1$-$C_4$-Alkylsulfonyl substituiertes $C_1$-$C_6$-Alkyl, für gegebenenfalls durch Fluor oder Chlor substituiertes Benzyl, für gegebenenfalls durch Fluor oder Chlor substituiertes $C_3$-$C_6$-Alkenyl oder $C_3$-$C_6$-Alkinyl, für gegebenenfalls durch Fluor, Chlor, Brom, $C_1$-$C_4$-Alkyl, $C_1$-$C_4$-Alkoxy, Trifluormethyl, Trifluormethoxy oder Trifluormethylthio substituiertes Phenyl, für gegebenenfalls durch Fluor und/ oder Chlor substituiertes $C_1$-$C_6$-Alkoxy, $C_3$-$C_6$-Alkenoxy, $C_3$-$C_6$-Alkinoxy oder Benzyloxy, für Amino, $C_1$-$C_4$-Alkylamino, Di-($C_1$-$C_4$-alkyl)-amino, Phenylamino, $C_1$-$C_4$-Alkyl-carbonyl-amino, $C_1$-$C_4$-Alkoxy-carbonylamino, $C_1$-$C_4$-Alkyl-sulfonylamino oder für gegebenenfalls durch Fluor, Chlor, Brom oder Methyl substituiertes Phenylsulfonylamino steht;

$R^1$ steht weiter vorzugsweise für den Rest

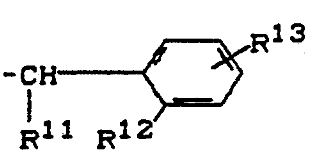

worin
$R^{11}$ für Wasserstoff oder $C_1$-$C_4$-Alkyl steht,
$R^{12}$ und $R^{13}$ gleich oder verschieden sind und für Wasserstoff, Fluor, Chlor, Brom, Nitro, Cyano, $C_1$-$C_4$-Alkyl (welches gegebenenfalls durch Fluor und/oder Chlor substituiert ist), $C_1C_4$-Alkoxy (welches gegebenenfalls durch Fluor und/oder Chlor substituiert ist), Carboxy, $C_1$-$C_4$-Alkoxy-carbonyl, $C_1$-$C_4$-Alkylsulfonyl oder Di-($C_1$-$C_4$-alkyl)-aminosulfonyl stehen;
$R^1$steht weiter vorzugsweise für den Rest

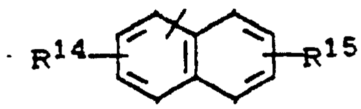

worin
$R^{14}$ und $R^{15}$ gleich oder verschieden sind und für Wasserstoff, Fluor, Chlor, Brom, Nitro, Cyano, $C_1$-$C_4$-Alkyl (welches gegebenenfalls durch Fluor und/oder Chlor substituiert ist) oder $C_1$-$C_4$-Alkoxy (welches gegebenenfalls durch Fluor und/oder Chlor substituiert ist), stehen;
$R^1$ steht weiter vorzugsweise für den Rest

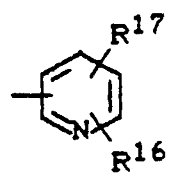

worin
$R^{16}$ und $R^{17}$gleich oder verschieden sind und für Wasserstoff, Fluor, Chlor, Brom, Nitro, Cyano, $C_1$-$C_4$-Alkyl (welches gegebenenfalls durch Fluor und/oder Chlor substituiert ist), $C_2$-$C_4$-Alkenyl (welches gegebenenfalls durch Fluor und/oder Chlor substituiert ist), $C_1$-$C_4$-Alkoxy (welches gegebenenfalls durch Fluor und/oder Chlor substituiert ist), für $C_1$-$C_4$-Alkylthio, $C_1$-$C_4$-Alkylsulfinyl oder $C_1$-$C_4$-Alkylsulfonyl (welche gegebenenfalls durch Fluor und/oder Chlor substituiert sind), sowie für Di-($C_1$-$C_4$-alkyl)-aminosulfonyl, $C_1$-$C_4$-Alkoxy-carbonyl, Dimethylaminocarbonyl oder Dioxolanyl stehen:
$R^1$ steht weiter vorzugsweise für den Rest

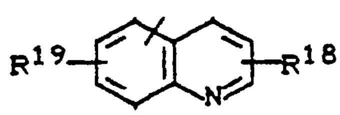

worin
$R^{18}$ und $R^{19}$ gleich oder verschieden sind und für Wasserstoff, Fluor, Chlor, Brom, $C_1$-$C_4$-Alkyl (welches gegebenenfalls durch Fluor und/oder Brom substituiert ist), $C_1$-$C_4$-Alkoxy (welches gegebenenfalls durch Fluor und/oder Chlor substituiert ist), für $C_1$-$C_4$-Alkylthio, $C_1$-$C_4$-Alkylsulfinyl oder $C_1$-$C_4$-Alkylsulfonyl (welche gegebenenfalls durch Fluor und/oder Chlor substituiert sind), oder für Di-($C_1$-$C_4$-alkyl)-aminosulfonyl stehen;
$R^1$ steht weiter vorzugsweise für den Rest

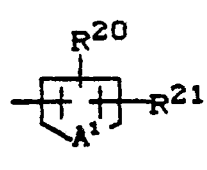

worin
$R^{20}$ und $R^{21}$ gleich oder verschieden sind und für Wasserstoff, Fluor, Chlor, Brom, Cyano, Nitro, $C_1$-$C_4$-Alkyl (welches gegebenenfalls durch Fluor, Chlor, $C_1$-$C_4$-Alkoxy und/oder $C_1$-$C_4$- Halogenalkoxy substituiert ist), $C_1$-$C_4$-Alkoxy (welches gegebenenfalls durch Fluor und/ oder Chlor substituiert ist), $C_1$-$C_4$-Alkylthio, $C_1$-$C_4$-Alkylsulfinyl oder $C_1$-$C_4$-Alkylsulfonyl (welches gegebenenfalls durch Fluor und/oder Chlor substituiert ist),

Di-($C_1$-$C_4$-alkyl)-amino-sulfonyl oder $C_1$-$C_4$-Alkoxy-carbonyl stehen, und
$A^1$ für Sauerstoff, Schwefel oder die Gruppierung N-$Z^1$ steht, wobei
$Z^1$ für Wasserstoff, $C_1$-$C_4$-Alkyl (welches gegebenenfalls durch Fluor, Chlor, Brom oder Cyano substituiert ist), $C_3$-$C_6$-Cycloalkyl, Benzyl, Phenyl (welches gegebenenfalls durch Fluor, Chlor, Brom oder Nitro substituiert ist), $C_1$-$C_4$-Alkylcarbonyl, $C_1$-$C_4$-Alkoxy-carbonyl oder Di-($C_1$-$C_4$-alkyl)-aminocarbonyl steht;
$R^1$ steht weiter vorzugsweise für den Rest

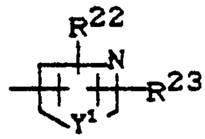

worin
$R^{22}$ und $R^{23}$ gleich oder verschieden sind und für Wasserstoff, $C_1$-$C_4$-Alkyl, Halogen, $C_1$-$C_4$-Alkoxycarbonyl, $C_1$-$C_4$-Alkoxy oder $C_1$-$C_4$-Halogenalkoxy stehen,
$Y^1$ für Schwefel oder die Gruppierung N-$R^{24}$ steht, wobei
$R^{24}$ für Wasserstoff oder $C_1$-$C_4$-Alkyl steht:
$R^1$ steht weiter vorzugsweise für den Rest

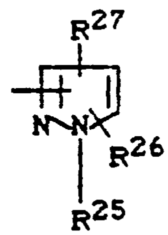

worin
$R^{25}$ für Wasserstoff $C_1$-$C_4$-Alkyl, Phenyl oder (Iso)Chinolinyl steht,
$R^{26}$ für Wasserstoff, Halogen, Cyano, Nitro, $C_1$-$C_4$-Alkyl (welches gegebenenfalls durch Fluor und/oder Chlor substituiert ist), $C_1$-$C_4$-Alkoxy (welches gegebenenfalls durch Fluor und/oder Chlor substituiert ist), Dioxolanyl oder $C_1$-$C_4$-Alkoxy-carbonyl steht und
$R^{27}$ für Wasserstoff, Halogen oder $C_1$-$C_4$-Alkyl steht:
$R^1$ steht weiter vorzugsweise für den Rest

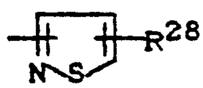

$R^{28}$ für Wasserstoff, Halogen, $C_1$-$C_4$-Alkyl oder $C_1$-$C_4$-Alkoxy-carbonyl steht, oder
für den Rest

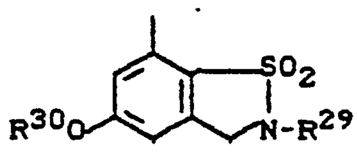

worin
$R^{29}$ für $C_1$-$C_4$-Alkyl steht und
$R^{30}$ für $C_1$-$C_4$-Alkyl steht, oder
für den Rest

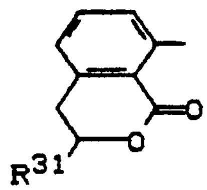

worin
$R^{31}$ für Wasserstoff oder Methyl steht;
weiter stehen in Formel (I) vorzugsweise
$R^2$ für Wasserstoff, Fluor, Chlor, Brom, $C_1$-$C_4$-Alkyl, Cyclopropyl, $C_1$-$C_4$-Halogenalkyl, $C_1$-$C_2$-Alkoxy-$C_1$-$C_2$-alkyl, $C_1$-$C_4$-Alkoxy, $C_1$-$C_4$-Halogenalkoxy, $C_1$-$C_4$-Alkylthio, $C_1$-$C_4$-Halogenalkylthio, Amino, $C_1$-$C_4$-Alkylamino, Dimethylamino oder Diethylamino,
X für Stickstoff oder eine CH-Gruppierung,
Y für Stickstoff oder die Gruppierung C-$R^3$, worin
$R^3$ für Wasserstoff, Fluor, Chlor, Brom, Cyano, Methyl, Formyl, Acetyl, Methoxycarbonyl oder Ethoxycarbonyl steht und
Z für Stickstoff oder die Gruppierung C-$R^4$, worin
$R^4$ für Wasserstoff, Fluor, Chlor, Brom, $C_1$-$C_4$-Alkyl, Cyclopropyl, $C_1$-$C_4$-Halogenalkyl, $C_1$-$C_4$- Alkoxy, $C_1$-$C_4$-Halogenalkoxy, $C_1$-$C_4$-Alkylthio, $C_1$-$C_4$-Alkylamino, Dimethylamino oder Diethylamino steht.

Insbesondere werden erfindungsgemäß die Verbindungen der Formel (I) verwendet, in welcher
$R^1$ für den Rest

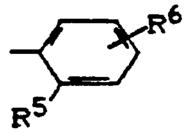

steht worin
$R^5$ für Wasserstoff Fluor, Chlor, Brom, Cyano, Methyl, Trifluormethyl, Methoxy, Difluormethoxy, Trifluormethoxy, $C_1$-$C_3$-Alkylthio, $C_1$-$C_3$-Alkylsulfinyl, $C_1$-$C_3$-Alkylsulfonyl, Dimethylaminosulfonyl, N-Methoxy-N-methylaminosulfonyl, Phenyl, Phenoxy oder $C_1$-$C_3$-Alkoxycarbonyl steht und
$R^6$ für Wasserstoff, Fluor oder Chlor steht; worin weiter
$R^1$ für den Rest

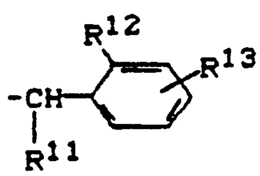

steht, worin
$R^{11}$ für Wasserstoff steht,
$R^{12}$ für Fluor, Chlor, Brom, Methyl, Methoxy, Difluormethoxy, Trifluormethoxy, Ethoxy, Methoxycarbonyl, Ethoxycarbonyl, Methylsulfonyl oder Dimethylaminosulfonyl steht und
$R^{13}$ für Wasserstoff, Fluor oder Chlor steht; worin weiter
$R^1$ für den Rest

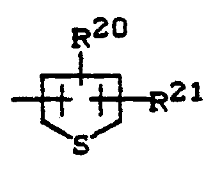

steht, worin
$R^{20}$ für Chlor, Methoxycarbonyl oder Ethoxycarbonyl steht und
$R^{21}$ für Wasserstoff oder Chlor steht; worin weiter
$R^1$ für den Rest

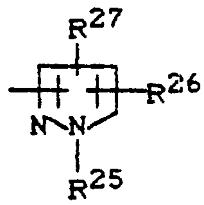

steht, worin
$R^{25}$ für Methyl oder Ethyl steht,
$R^{26}$ für Chlor, Methoxycarbonyl oder Ethoxycarbonyl steht und
$R^{27}$ für Wasserstoff oder Chlor steht,
in welcher weiter
$R^2$ für Wasserstoff, Fluor, Chlor, Brom, Methyl, Ethyl, Cyclopropyl, Trifluormethyl Methoxy, Ethoxy, Difluormethoxy, Methylthio, Ethylthio, Amino, Methylamino, Ethylamino, Dimethylamino oder Diethylamino steht,
X für Stickstoff oder eine CH-Gruppierung steht,
Y für Stickstoff oder eine $CR^5$-Gruppierung steht, worin
$R^5$ für Wasserstoff, Fluor, Chlor oder Methyl steht, und
Z für Stickstoff oder eine $CR^6$-Gruppierung steht, worin
$R^6$ für Wasserstoff, Fluor, Chlor, Brom, Methyl, Ethyl, Trifluormethyl; Methoxy, Ethoxy, Propoxy, Isopropoxy, Difluormethoxy, Methylthio, Ethylthio, Methylamino, Ethylamino, Dimethylamino oder Diethylamino steht.

Beispiele für besonders bevorzugte, erfindungsgemäß zu verwendende Verbindungen der Formel (I) sind in der nachstehenden Tabelle 1 aufgeführt.

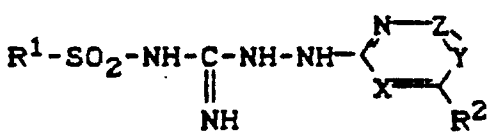

(I)

Tabelle 1: Beispiele für die Verbindungen der Formel (I)

| $R^1$ | $R^2$ | X | Y | Z |
|---|---|---|---|---|
| 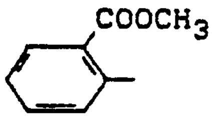 | $CH_3$ | N | CH | $C-CH_3$ |
| 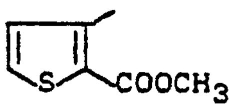 | $CH_3$ | N | CH | $C-CH_3$ |
| 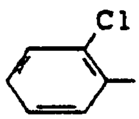 | $CH_3$ | N | CH | $C-CH_3$ |
| 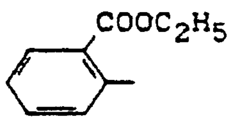 | $CH_3$ | N | CH | $C-CH_3$ |
| 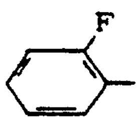 | $CH_3$ | N | CH | $C-CH_3$ |
| 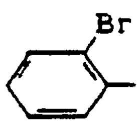 | $CH_3$ | N | CH | $C-CH_3$ |
| 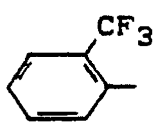 | $CH_3$ | N | CH | $C-CH_3$ |
| 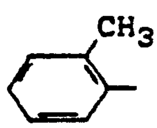 | $CH_3$ | N | CH | $C-CH_3$ |
| 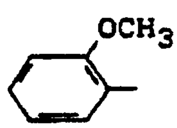 | $CH_3$ | N | CH | $C-CH_3$ |

Tabelle 1 - Fortsetzung

| $R^1$ | $R^2$ | X | Y | Z |
|---|---|---|---|---|
| 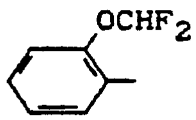 | $CH_3$ | N | CH | $C-CH_3$ |
| 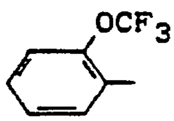 | $CH_3$ | N | CH | $C-CH_3$ |
| 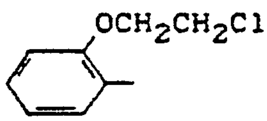 | $CH_3$ | N | CH | $C-CH_3$ |
| 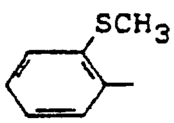 | $CH_3$ | N | CH | $C-CH_3$ |
| 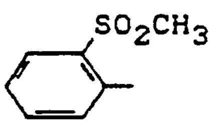 | $CH_3$ | N | CH | $C-CH_3$ |
| 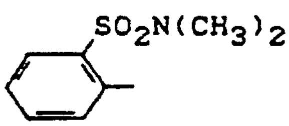 | $CH_3$ | N | CH | $C-CH_3$ |
| 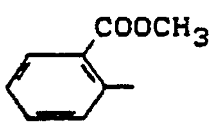 | $CH_3$ | N | CH | $C-OCH_3$ |
| 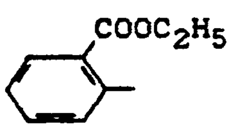 | $CH_3$ | N | CH | $C-OCH_3$ |

<u>Tabelle 1</u> - Fortsetzung

| $R^1$ | $R^2$ | X | Y | Z |
|---|---|---|---|---|
| 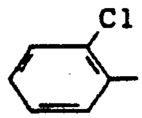 | $CH_3$ | N | CH | $C-OCH_3$ |
| 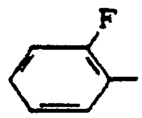 | $CH_3$ | N | CH | $C-OCH_3$ |
| 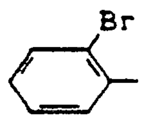 | $CH_3$ | N | CH | $C-OCH_3$ |
| 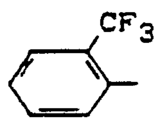 | $CH_3$ | N | CH | $C-OCH_3$ |
| 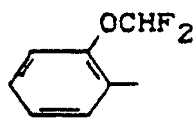 | $CH_3$ | N | CH | $C-OCH_3$ |
| 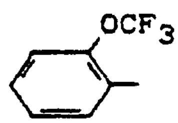 | $CH_3$ | N | CH | $C-OCH_3$ |
| 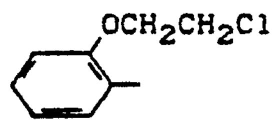 | $CH_3$ | N | CH | $C-OCH_3$ |
| 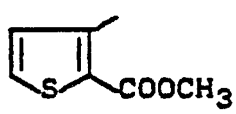 | $CH_3$ | N | CH | $C-OCH_3$ |
| 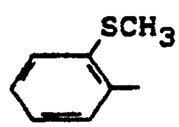 | $CH_3$ | N | CH | $C-OCH_3$ |

Tabelle 1 - Fortsetzung

| $R^1$ | $R^2$ | X | Y | Z |
|---|---|---|---|---|
| 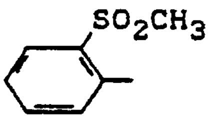 | $CH_3$ | N | CH | $C-OCH_3$ |
| 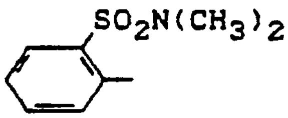 | $CH_3$ | N | CH | $C-OCH_3$ |
| 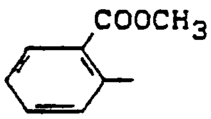 | $OCH_3$ | N | CH | $C-OCH_3$ |
| 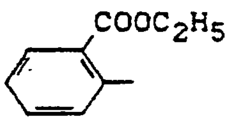 | $OCH_3$ | N | CH | $C-OCH_3$ |
| 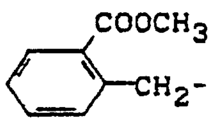 | $OCH_3$ | N | CH | $C-OCH_3$ |
| 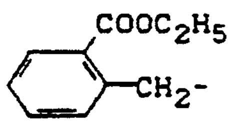 | $OCH_3$ | N | CH | $C-OCH_3$ |
| 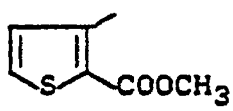 | $OCH_3$ | N | CH | $C-OCH_3$ |
| 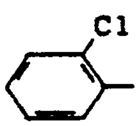 | $OCH_3$ | N | CH | $C-OCH_3$ |
| 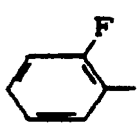 | $OCH_3$ | N | CH | $C-OCH_3$ |

Tabelle 1 - Fortsetzung

| $R^1$ | $R^2$ | X | Y | Z |
|---|---|---|---|---|
| 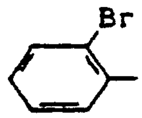 | $OCH_3$ | N | CH | C-$OCH_3$ |
| 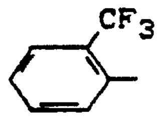 | $OCH_3$ | N | CH | C-$OCH_3$ |
| 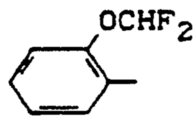 | $OCH_3$ | N | CH | C-$OCH_3$ |
| 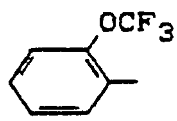 | $OCH_3$ | N | CH | C-$OCH_3$ |
| 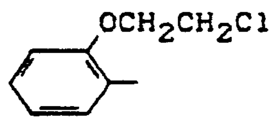 | $OCH_3$ | N | CH | C-$OCH_3$ |
| 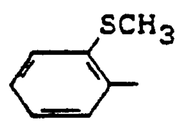 | $OCH_3$ | N | CH | C-$OCH_3$ |
| 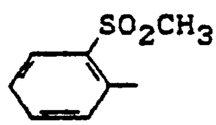 | $OCH_3$ | N | CH | C-$OCH_3$ |
| 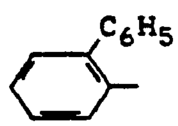 | $OCH_3$ | N | CH | C-$OCH_3$ |

Tabelle 1 - Fortsetzung

| $R^1$ | $R^2$ | X | Y | Z |
|---|---|---|---|---|
| 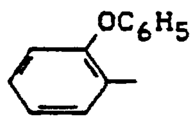 | $OCH_3$ | N | CH | $C-OCH_3$ |
| 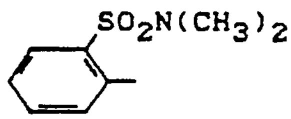 | $OCH_3$ | N | CH | $C-OCH_3$ |
| 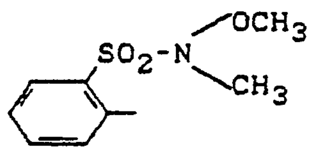 | $OCH_3$ | N | CH | $C-OCH_3$ |
| 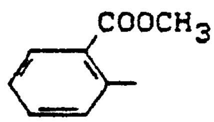 | $CF_3$ | N | CH | $C-OCH_3$ |
| 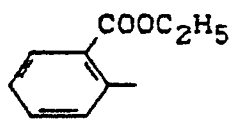 | $CF_3$ | N | CH | $C-OCH_3$ |
| 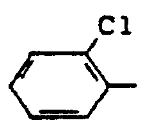 | $CF_3$ | N | CH | $C-OCH_3$ |
| 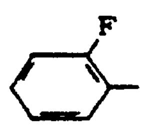 | $CF_3$ | N | CH | $C-OCH_3$ |
| 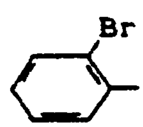 | $CF_3$ | N | CH | $C-OCH_3$ |

<u>Tabelle 1</u> - Fortsetzung

| $R^1$ | $R^2$ | X | Y | Z |
|---|---|---|---|---|
| 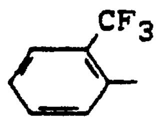 | $CF_3$ | N | CH | $C-OCH_3$ |
| 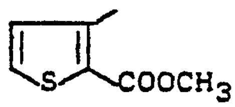 | $CF_3$ | N | CH | $C-OCH_3$ |
| 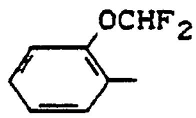 | $CF_3$ | N | CH | $C-OCH_3$ |
| 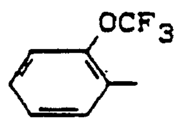 | $CF_3$ | N | CH | $C-OCH_3$ |
| 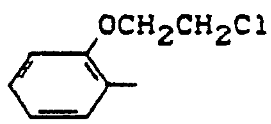 | $CF_3$ | N | CH | $C-OCH_3$ |
| 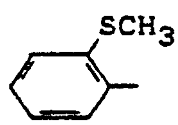 | $CF_3$ | N | CH | $C-OCH_3$ |
| 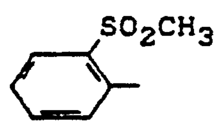 | $CF_3$ | N | CH | $C-OCH_3$ |
| 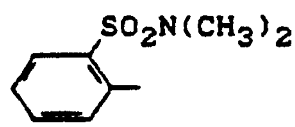 | $CF_3$ | N | CH | $C-OCH_3$ |
| 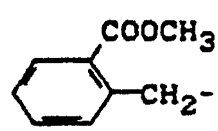 | $CF_3$ | N | CH | $C-OCH_3$ |

Tabelle 1 - Fortsetzung

| $R^1$ | $R^2$ | X | Y | Z |
|---|---|---|---|---|
| 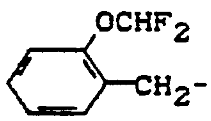 | $CF_3$ | N | CH | $C-OCH_3$ |
| 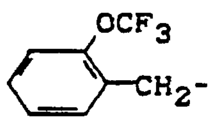 | $CF_3$ | N | CH | $C-OCH_3$ |
| 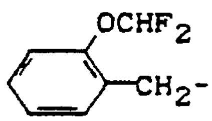 | $CH_3$ | N | CH | $C-OCH_3$ |
| 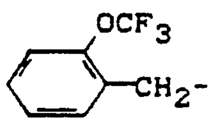 | $CH_3$ | N | CH | $C-OCH_3$ |
| 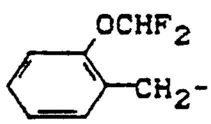 | $OCH_3$ | N | CH | $C-OCH_3$ |
| 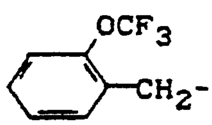 | $OCH_3$ | N | CH | $C-OCH_3$ |
| 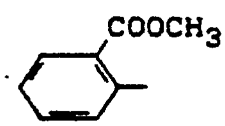 | $OCH_3$ | N | CH | C-Cl |
| 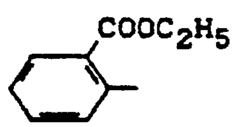 | $OCH_3$ | N | CH | C-Cl |

Tabelle 1 - Fortsetzung

| $R^1$ | $R^2$ | X | Y | Z |
|---|---|---|---|---|
| 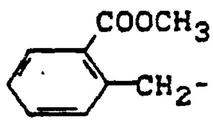 | $OCH_3$ | N | CH | C-Cl |
| 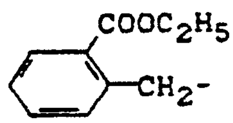 | $OCH_3$ | N | CH | C-Cl |
| 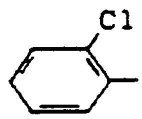 | $OCH_3$ | N | CH | C-Cl |
| 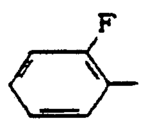 | $OCH_3$ | N | CH | C-Cl |
| 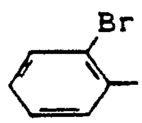 | $OCH_3$ | N | CH | C-Cl |
| 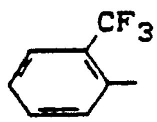 | $OCH_3$ | N | CH | C-Cl |
| 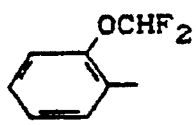 | $OCH_3$ | N | CH | C-Cl |
| 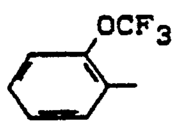 | $OCH_3$ | N | CH | C-Cl |

<u>Tabelle 1</u> - Fortsetzung

| $R^1$ | $R^2$ | X | Y | Z |
|---|---|---|---|---|
| 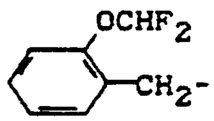 | $OCH_3$ | N | CH | C-Cl |
| 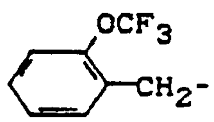 | $OCH_3$ | N | CH | C-Cl |
| 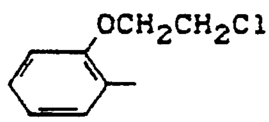 | $OCH_3$ | N | CH | C-Cl |
| 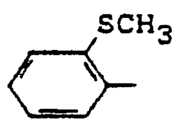 | $OCH_3$ | N | CH | C-Cl |
| 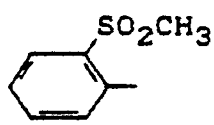 | $OCH_3$ | N | CH | C-Cl |
| 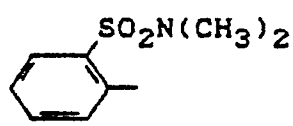 | $OCH_3$ | N | CH | C-Cl |
| 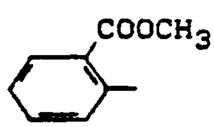 | $CH_3$ | N | CH | C-$OCHF_2$ |
| 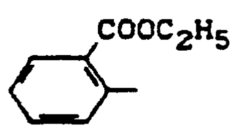 | $CH_3$ | N | CH | C-$OCHF_2$ |

Tabelle 1 - Fortsetzung

| $R^1$ | $R^2$ | X | Y | Z |
|---|---|---|---|---|
| 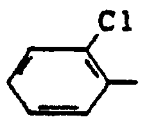 | $CH_3$ | N | CH | $C\text{-}OCHF_2$ |
| 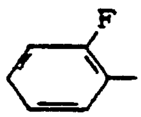 | $CH_3$ | N | CH | $C\text{-}OCHF_2$ |
| 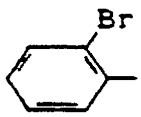 | $CH_3$ | N | CH | $C\text{-}OCHF_2$ |
| 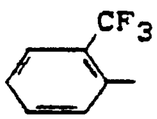 | $CH_3$ | N | CH | $C\text{-}OCHF_2$ |
| 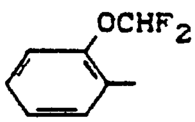 | $CH_3$ | N | CH | $C\text{-}OCHF_2$ |
| 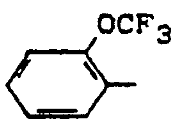 | $CH_3$ | N | CH | $C\text{-}OCHF_2$ |
| 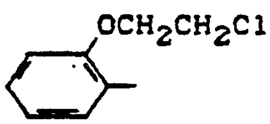 | $CH_3$ | N | CH | $C\text{-}OCHF_2$ |
| 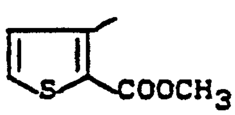 | $CH_3$ | N | CH | $C\text{-}OCHF_2$ |

Tabelle 1 - Fortsetzung

| $R^1$ | $R^2$ | X | Y | Z |
|---|---|---|---|---|
| 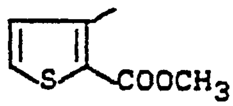 | $OCH_3$ | N | CH | C-Cl |
| 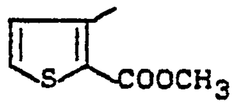 | $OCH_3$ | N | CH | $C-OCHF_2$ |
| 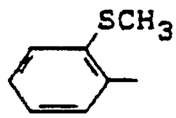 | $CH_3$ | N | CH | $C-OCHF_2$ |
| 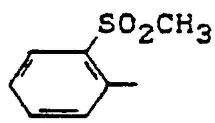 | $CH_3$ | N | CH | $C-OCHF_2$ |
| 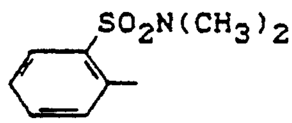 | $CH_3$ | N | CH | $C-OCHF_2$ |
| 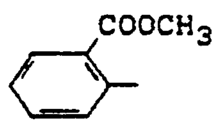 | $OCH_3$ | N | CH | $C-OCHF_2$ |
| 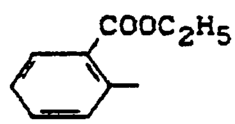 | $OCH_3$ | N | CH | $C-OCHF_2$ |
| 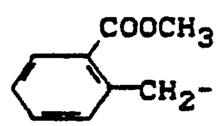 | $OCH_3$ | N | CH | $C-OCHF_2$ |
| 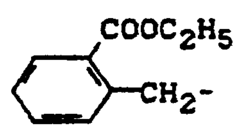 | $OCH_3$ | N | CH | $C-OCHF_2$ |

Tabelle 1 - Fortsetzung

| $R^1$ | $R^2$ | X | Y | Z |
| --- | --- | --- | --- | --- |
| 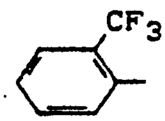 | $OCH_3$ | N | CH | C-$OCHF_2$ |
| 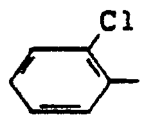 | $OCH_3$ | N | CH | C-$OCHF_2$ |
| 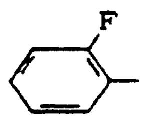 | $OCH_3$ | N | CH | C-$OCHF_2$ |
| 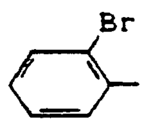 | $OCH_3$ | N | CH | C-$OCHF_2$ |
| 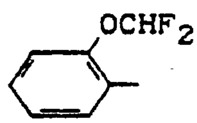 | $OCH_3$ | N | CH | C-$OCHF_2$ |
| 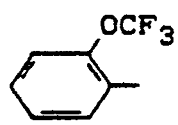 | $OCH_3$ | N | CH | C-$OCHF_2$ |
| 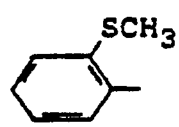 | $OCH_3$ | N | CH | C-$OCHF_2$ |
| 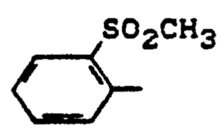 | $OCH_3$ | N | CH | C-$OCHF_2$ |

Tabelle 1 - Fortsetzung

| $R^1$ | $R^2$ | X | Y | Z |
|---|---|---|---|---|
| 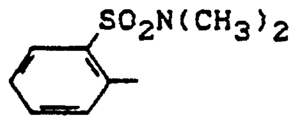 | $OCH_3$ | N | CH | $C\text{-}OCHF_2$ |
| 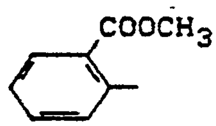 | $OCHF_2$ | N | CH | $C\text{-}OCHF_2$ |
| 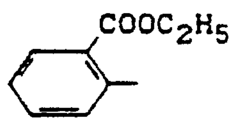 | $OCHF_2$ | N | CH | $C\text{-}OCHF_2$ |
| 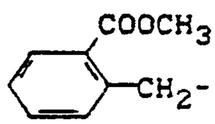 | $OCHF_2$ | N | CH | $C\text{-}OCHF_2$ |
| 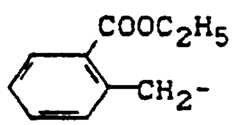 | $OCHF_2$ | N | CH | $C\text{-}OCHF_2$ |
| 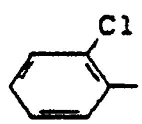 | $OCHF_2$ | N | CH | $C\text{-}OCHF_2$ |
| 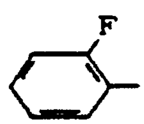 | $OCHF_2$ | N | CH | $C\text{-}OCHF_2$ |
| 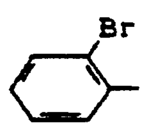 | $OCHF_2$ | N | CH | $C\text{-}OCHF_2$ |

Tabelle 1 - Fortsetzung

| $R^1$ | $R^2$ | X | Y | Z |
|---|---|---|---|---|
| 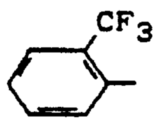 | $OCHF_2$ | N | CH | $C-OCHF_2$ |
| 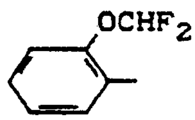 | $OCHF_2$ | N | CH | $C-OCHF_2$ |
| 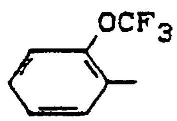 | $OCHF_2$ | N | CH | $C-OCHF_2$ |
| 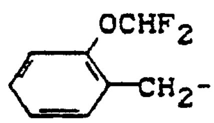 | $OCHF_2$ | N | CH | $C-OCHF_2$ |
| 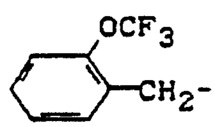 | $OCHF_2$ | N | CH | $C-OCHF_2$ |
| 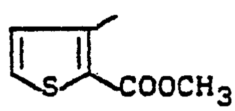 | $OCHF_2$ | N | CH | $C-OCHF_2$ |
| 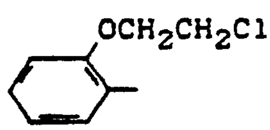 | $OCHF_2$ | N | CH | $C-OCHF_2$ |
| 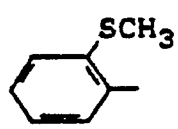 | $OCHF_2$ | N | CH | $C-OCHF_2$ |

<u>Tabelle 1</u> - Fortsetzung

| $R^1$ | $R^2$ | X | Y | Z |
|---|---|---|---|---|
| 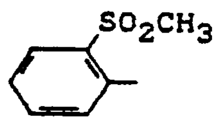 | $OCHF_2$ | N | CH | $C-OCHF_2$ |
| 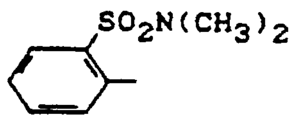 | $OCHF_2$ | N | CH | $C-OCHF_2$ |
| 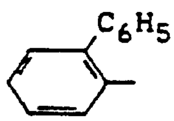 | $OCHF_2$ | N | CH | $C-OCHF_2$ |
| 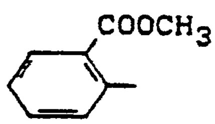 | $CH_3$ | N | N | $C-CH_3$ |
| 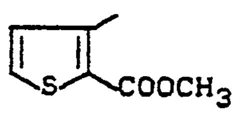 | $CH_3$ | N | N | $C-CH_3$ |
| 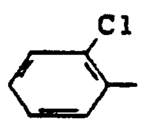 | $CH_3$ | N | N | $C-CH_3$ |
| 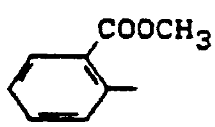 | $CH_3$ | N | N | $C-OCH_3$ |
| 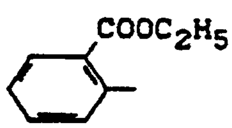 | $CH_3$ | N | N | $C-OCH_3$ |
| 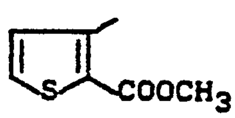 | $CH_3$ | N | N | $C-OCH_3$ |

<u>Tabelle 1</u> - Fortsetzung

| $R^1$ | $R^2$ | X | Y | Z |
|---|---|---|---|---|
| 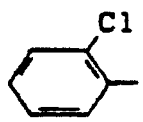 | $CH_3$ | N | N | $C-OCH_3$ |
| 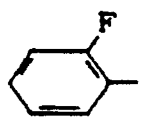 | $CH_3$ | N | N | $C-OCH_3$ |
| 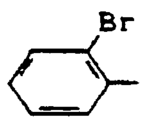 | $CH_3$ | N | N | $C-OCH_3$ |
| 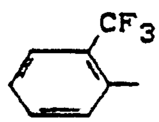 | $CH_3$ | N | N | $C-OCH_3$ |
| 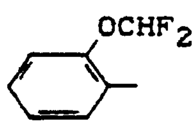 | $CH_3$ | N | N | $C-OCH_3$ |
| 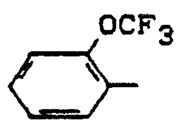 | $CH_3$ | N | N | $C-OCH_3$ |
| 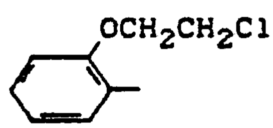 | $CH_3$ | N | N | $C-OCH_3$ |
| 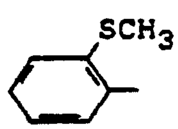 | $CH_3$ | N | N | $C-OCH_3$ |
| 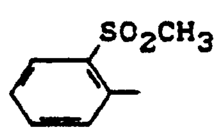 | $CH_3$ | N | N | $C-OCH_3$ |

Tabelle 1 - Fortsetzung

| $R^1$ | $R^2$ | X | Y | Z |
|---|---|---|---|---|
| 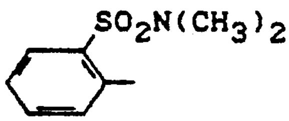 | $CH_3$ | N | N | $C-OCH_3$ |
| 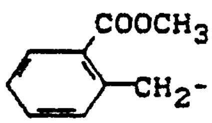 | $CH_3$ | N | N | $C-OCH_3$ |
| 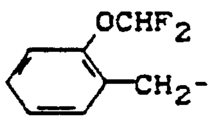 | $CH_3$ | N | N | $C-OCH_3$ |
| 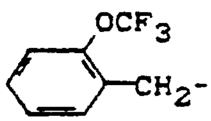 | $CH_3$ | N | N | $C-OCH_3$ |
| 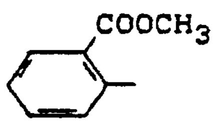 | $OCH_3$ | N | N | $C-OCH_3$ |
| 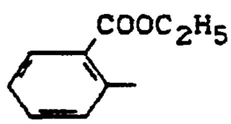 | $OCH_3$ | N | N | $C-OCH_3$ |
| 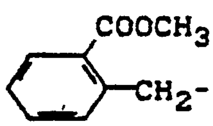 | $OCH_3$ | N | N | $C-OCH_3$ |
| 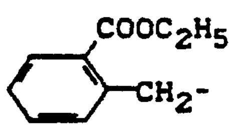 | $OCH_3$ | N | N | $C-OCH_3$ |

<u>Tabelle 1</u> - Fortsetzung

| $R^1$ | $R^2$ | X | Y | Z |
|---|---|---|---|---|
| 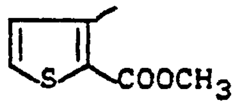 | $OCH_3$ | N | N | $C-OCH_3$ |
| 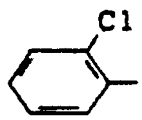 | $OCH_3$ | N | N | $C-OCH_3$ |
| 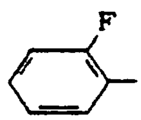 | $OCH_3$ | N | N | $C-OCH_3$ |
| 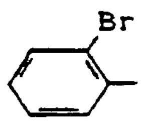 | $OCH_3$ | N | N | $C-OCH_3$ |
| 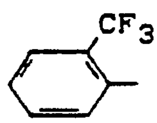 | $OCH_3$ | N | N | $C-OCH_3$ |
| 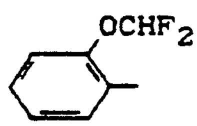 | $OCH_3$ | N | N | $C-OCH_3$ |
| 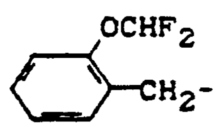 | $OCH_3$ | N | N | $C-OCH_3$ |
| 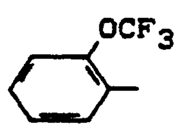 | $OCH_3$ | N | N | $C-OCH_3$ |
| 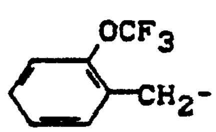 | $OCH_3$ | N | N | $C-OCH_3$ |

<u>Tabelle 1</u> - Fortsetzung

| $R^1$ | $R^2$ | X | Y | Z |
|---|---|---|---|---|
| 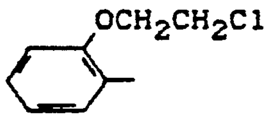 | $OCH_3$ | N | N | $C-OCH_3$ |
| 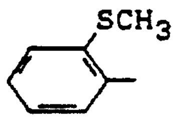 | $OCH_3$ | N | N | $C-OCH_3$ |
| 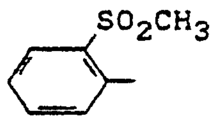 | $OCH_3$ | N | N | $C-OCH_3$ |
| 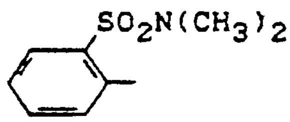 | $OCH_3$ | N | N | $C-OCH_3$ |
| 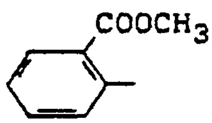 | $C_2H_5$ | N | N | $C-OCH_3$ |
| 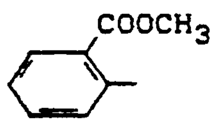 | $CH_3$ | N | N | $C-OC_2H_5$ |
| 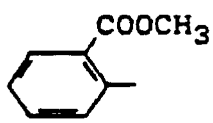 | $OCH_3$ | N | N | $C-NHC_2H_5$ |
| 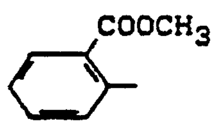 | $OC_2H_5$ | N | N | $C-NHCH_3$ |

Tabelle 1 - Fortsetzung

| $R^1$ | $R^2$ | X | Y | Z |
|---|---|---|---|---|
| 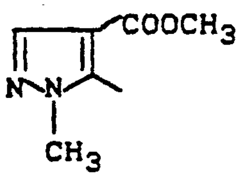 | $OCH_3$ | N | CH | $C-OCH_3$ |
| 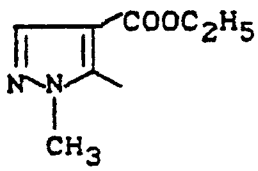 | $OCH_3$ | N | CH | $C-OCH_3$ |
| 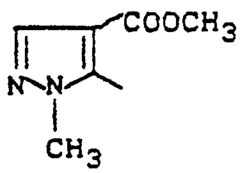 | $OCH_3$ | N | N | $C-OCH_3$ |
| 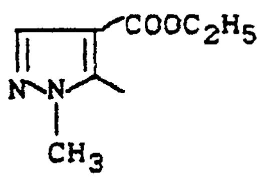 | $OCH_3$ | N | N | $C-OCH_3$ |
| 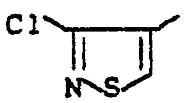 | $OCH_3$ | N | CH | $C-OCH_3$ |
| 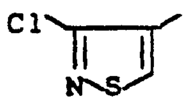 | $OCH_3$ | N | N | $C-OCH_3$ |
| 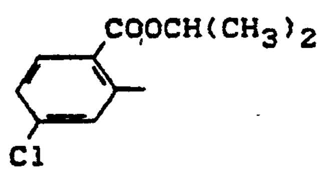 | $OCH_3$ | N | N | $C-OCH_3$ |
| 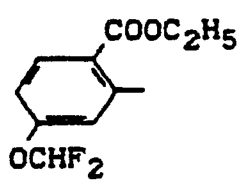 | $OCH_3$ | N | CH | $C-OCH_3$ |

Tabelle 1 - Fortsetzung

| $R^1$ | $R^2$ | X | Y | Z |
|---|---|---|---|---|
| 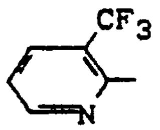 | $OCH_3$ | N | CH | $C-OCH_3$ |
| 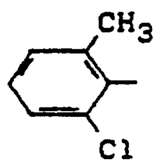 | $OCH_3$ | N | CH | $C-OCH_3$ |
| 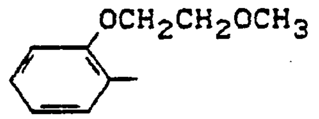 | $OCH_3$ | N | CH | $C-OCH_3$ |
| 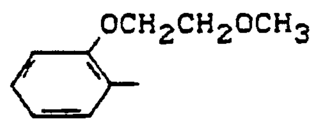 | $OCH_3$ | N | N | $C-OCH_3$ |

Die sulfonylierten Aminoguanidine der Formel (I) sind Gegenstand einer älteren, nicht vorveröffentlichten Patentanmeldung der Anmelderin (vgl. DE-P 38 25 867 vom 29.7.1988).

Man erhält die Verbindungen der Formel (I), wenn man die dazu isomeren Verbindungen der allgemeinen Formel (II)

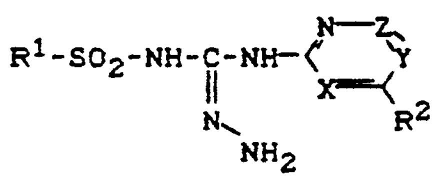

(II)

in welcher
$R^1$, $R^2$, X, Y und Z die oben angegebenen Bedeutungen haben,
- oder Tautomere zu den Verbindungen der Formel (II) oder Gemische aus Verbindungen der Formel (II) und den jeweils möglichen Tautomeren -
nach üblichen Isomerisierungsmethoden, beispielsweise durch Verrühren mit polaren Lösungsmitteln, wie z.B. Methanol, Ethanol, Propanol, Isopropanol, Dimethylsulfoxid und/oder Wasser, gegebenenfalls in Gegenwart katalytischer Mengen von Säuren, wie z.B. Salzsäure oder Schwefelsäure, bei Temperaturen zwischen 0 °C und 150 °C umlagert.

Zur Herstellung der Verbindungen der Formel (I) können gegebenenfalls auch die Isomeren der Formel (II) wie nachstehend beschrieben erzeugt und in situ, d.h. ohne Zwischenisolierung, umgelagert werden.

Die Verbindungen der Formel (II) sind teilweise bekannt (vgl. EP-A 224 078, US-P 4 725 303) und teilweise Gegenstand einer nicht vorveröffentlichten DE-Patentanmeldung (P 38 18 040 vom 27.5.1988).

Man erhält die Verbindungen der Formel (II), wenn man Sulfonylverbindungen der allgemeinen Formel (III)

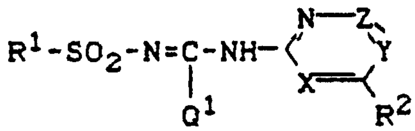

(III)

in welcher
$R^1$, $R^2$, X, Y und Z die oben angegebenen Bedeutungen haben und
$Q^1$ für Halogen oder eine der nachstehend angegebenen Abgangsgruppen

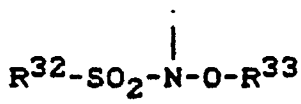

oder $-Q^2-R^{34}$ steht, worin
$R^{32}$ die oben für $R^1$ angegebene Bedeutung hat, aber nicht in jedem Einzelfall mit $R^1$ identisch sein muß,
$R^{33}$ für Alkyl, Alkenyl oder Aralkyl steht,
$R^{34}$ für jeweils gegebenenfalls substituiertes Alkyl, Aralkyl oder Aryl steht und
$Q^2$ für Sauerstoff oder Schwefel steht,
mit Hydrazin oder einem Hydrazin-Wasser- oder Hydrazin-Säure-Addukt,
gegebenenfalls in Gegenwart eines Säureakzeptors, wie z. B. Kaliumcarbonat, und gegebenenfalls in Gegenwart eines Verdünnungsmittels, wie z. B. Methanol, Ethanol und/oder Wasser, bei Temperaturen zwischen -20 °C und +100 °C umsetzt.

Die Verbindungen der Formel (III) sind bekannt und/oder können nach an sich bekannten Verfahren hergestellt werden (vgl. EP-A 5986, EP-A 24215, EP-A 121 082, EP-A 172 957, EP-A 173 321, EP-A 173 956, EP-A 224 078, DE-OS 36 34 928, DE-OS 36 34 929).

Die erfindungsgemäß zu verwendenden Verbindungen sind besonders gut geeignet zur selektiven Bekämpfung von dikotylen Unkräutern in monokotylen Kulturen, wie z.B. Weizen, sowohl im Vorauflauf- als auch im Nachauflauf-Verfahren.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Qranulate aus organischem Material wie Sägemehl, Kokosnußschalen, Maiskolben und Tabakstengeln: als Emulgier- und/oder schaumerzeugende Mittel kommen in Frage: z.B. nichtionogene und anionische Emulgatoren, wie Polyoxyethylen-Fettsäure-Ester, Poly oxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxide, Titanoxid, Ferrocyanblau und organische Farbstoffe, wie Alizarin-, Azo- und Metallphthalocyaninfarbstoffe und Spurennäherstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1- Amino-6-ethylthio-3-(2,2-dimethylpropyl)-1,3,5-triazin-2,4(1H,3H)-dion (AMETHYDIONE) oder N-(2-Benzthiazolyl)-N,N′-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, in Frage; ferner auch 2,4-Dichlorphenoxyessigsäure (2,4-D); 4-(2,4-Dichlorphenoxy)-buttersäure (2,4-DB); 2,4-Dichlorphenoxypropionsäure (2,4-DP); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); 2-Chlor-N-{[(4-methoxy-6-methyl-1,3,5-triazin-2-yl)-amino]-carbonyl}-benzolsulfonamid (CHLORSULFURON); N,N-Dimethyl-N′-(3-chlor-4-methylphenyl)-harnstoff (CHLORTOLURON); 2-[4-(2,4-Dichlorphenoxy)-phenoxy]-propionsäure, deren Methyl-oder deren Ethylester (DICLOFOP); 4-Amino-6-t-butyl-3-ethylthio-1,2,4-triazin-5-(4H)-on (ETHIOZIN); 2-{4-[(6-Chlor-2-benzoxazolyl)-oxy]-phenoxy}-propensäure, deren Methyl-oder deren Ethylester (FENOXAPROP); [(4-Amino-3,5-dichlor-6-fluor-2-pyridinyl)-oxy]-essigsäure bzw. deren 1-Methylheptylester (FLUROXYPYR); Methyl-2-[4,5-dihydro-4-methyl-4-(1-methylethyl)-5-oxo-1H-imidazol-2-yl]-4(5)-methylbenzoat (IMAZAMETHABENZ); 3,5-Diiod-4-hydroxybenzonitril (IOXYNIL); N,N-Dimethyl-N′-(4-isopropylphenyl)-harnstoff (ISOPROTURON); (2-Methyl-4-chlorphenoxy)-essigsäure (MCPA); (4-Chlor-2-methylphenoxy)-propionsäure (MCPP); N-Methyl-2-(1,3-benzthiazol-2-yloxy)-acetanilid (MEFENACET); 2-{[[((4-Methoxy-6- methyl-1,3,5-triazin-2-yl)-amino)-carbonyl]-amino]-sulfonyl}-benzoesäure oder deren Methylester (METSULFURON): N-(1-Ethylpropyl)-3,4-dimethyl-2,6-dinitroanilin (PENDIMETHALIN); O-(6-Chlor-3-phenyl-pyridazin-4-yl)-S-octyl-thiocarbonat (PYRIDATE); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-sulfonyl]-thiophen-2-carbonsäure-methylester (THIAMETURON) und N,N-Diisopropyl-S-(2,3,3-trichlorallyl)-thiolcarbamat (TRIALLATE).

Einige Mischungen zeigen überraschenderweise auch synergistische Wirkung.

Auch eine Mischung mit anderen bekannten Wirkstoffen, wie Fungiziden, Insektiziden, Akariziden,

Hematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Giesen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

Anwendungsbeispiele:

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
0 % = keine Wirkung (wie unbehandelte Kontrolle)
100 % = totale Vernichtung

In diesem Test zeigen z.B. die Verbindungen gemäß den Herstellungsbeispielen 5, 19 und 29 bei guter Selektivität in Weizen starke Wirkung gegen Unkräuter, wie z.B. Chenopodium, Galinsoga, Matricaria, Portulaca, Stellaria und Viola.

**Tabelle : A** Pre-emergence-Test / Gewächshaus

| Wirkstoff | Aufwandmenge (g/ha) | Weizen | Chenopodium | Galinsoga | Matricaria | Potulaca | Stellaria | Viola |
|---|---|---|---|---|---|---|---|---|
| 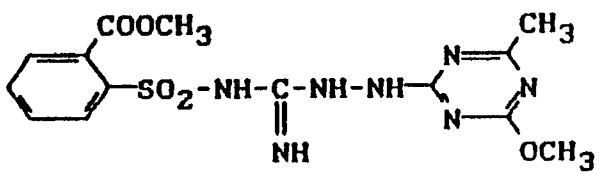 (5) | 60 | 0 | 80 | 90 | 95 | 90 | 95 | 80 |
| 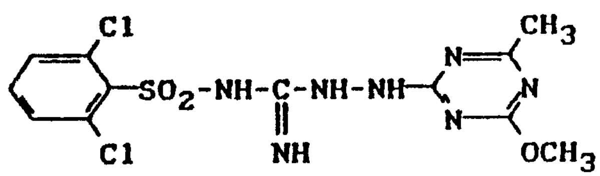 (19) | 60 | 0 | 90 | 80 | 80 | 95 | 90 | 70 |
| 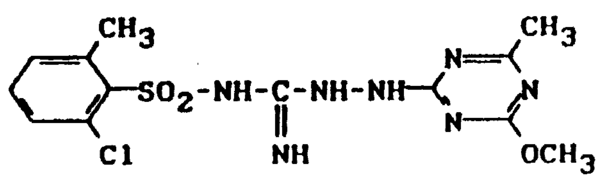 (29) | 60 | 30 | 95 | 80 | 95 | 95 | 90 | 80 |

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben, so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:
0 % = keine Wirkung (wie unbehandelte Kontrolle)
100 % = totale Vernichtung

In diesem Test zeigen z.B. die Verbindungen gemäß den Herstellungsbeispielen 5, 12, 19, 25, 26, 29, 34, 49 und 61 bei guter Selektivität in Weizen starke Wirkung gegen Unkräuter, wie z.B. Helianthus, Ipomea, Sinapis und Stellaria.

**Tabelle B :** Post-emergence-Test / Gewächshaus

| Wirkstoff | Aufwandmenge (g/ha) | Weizen | Helianthus | Ipomoea | Sinapis | Stellaria |
|---|---|---|---|---|---|---|
| 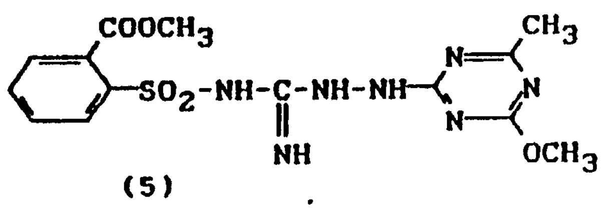 (5) | 125 | 0 | 100 | 100 | 90 | 100 |
| 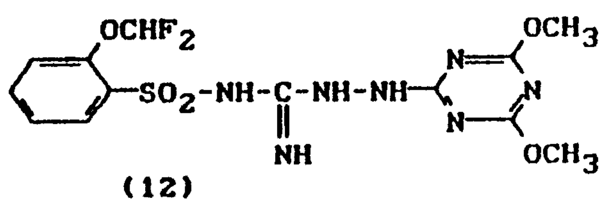 (12) | 125 | 0 | 100 | 90 | 95 | 95 |
| 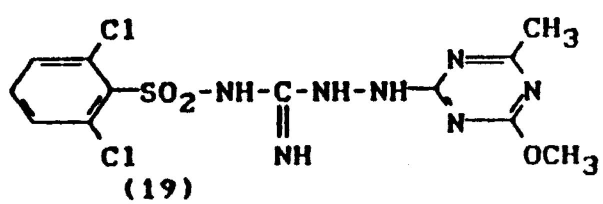 (19) | 125 | 0 | 100 | 90 | 95 | 95 |

**Tabelle B :** Post-emergence-Test / Gewächshaus - Fortsetzung

| Wirkstoff | Aufwandmenge (g/ha) | Weizen | Helianthus | Ipomoea | Sinapis | Stellaria |
|---|---|---|---|---|---|---|
| 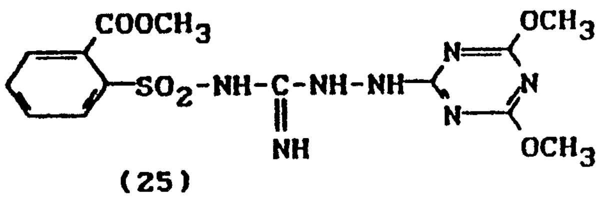 (25) | 125 | 0 | 100 | 90 | 100 | 95 |
| 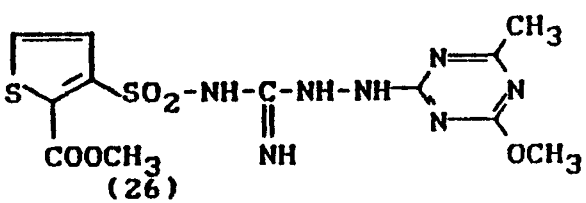 (26) | 125 | 0 | 100 | 90 | 90 | 90 |
| 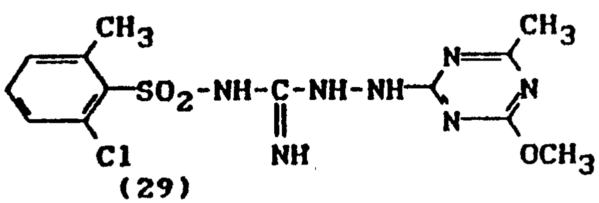 (29) | 125 | 0 | 100 | 90 | 90 | 100 |

**Tabelle B :** Post-emergence-Test / Gewächshaus - Fortsetzung

| Wirkstoff | Aufwandmenge (g/ha) | Weizen | Helianthus | Ipomoea | Sinapis | Stellaria |
|---|---|---|---|---|---|---|
| 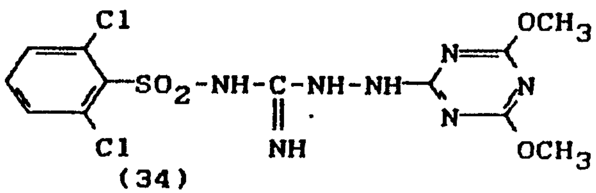 (34) | 125 | 0 | 95 | 50 | 90 | 80 |
| 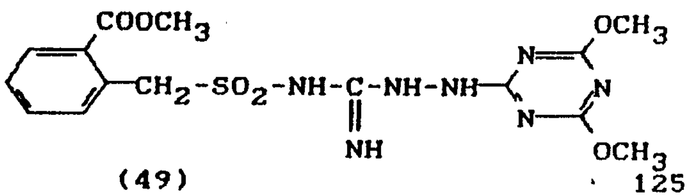 (49) | 125 | 10 | 100 | 90 | 95 | 95 |
| 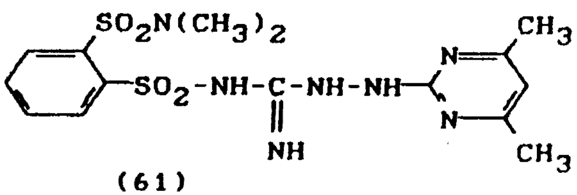 (61) | 125 | 0 | 80 | 80 | 90 | - |

Herstellungsbeispiele:

Beispiel 1

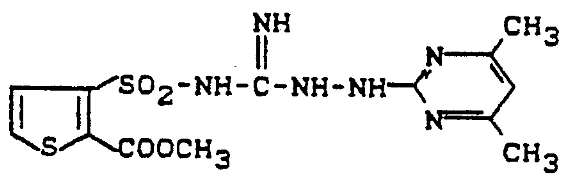

Eine Lösung von 3,8 g (0,01 Mol) N′-(4,6-Dimethyl-pyrimidin-2-yl)-N″-amino-N‴-(2-methoxycarbonyl-thien-3-yl-sulfonyl)-guanidin in 100 ml Ethanol wird 15 Stunden unter Rückfluß zum Sieden erhitzt. Nach Abkühlen des Reaktionsgemisches auf 0 °C wird das kristallin angefallene Produkt durch Absaugen isoliert.

Man erhält 2,2 g (58 % der Theorie) N′-(4,6-Dimethylpyrimidin-2-yl-amino)-N″-(2-methoxycarbonyl-thien-3-yl-sulfonyl)-guanidin vom Schmelzpunkt 213 °C.

Beispiel 2

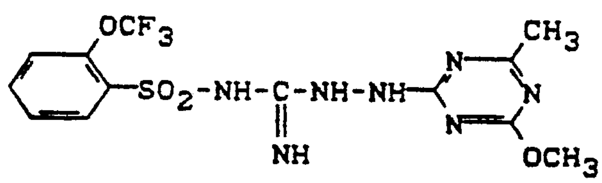

Eine Mischung aus 8,8 g (0,02 Mol) N′-(4-Methyl-6-methoxy-s-triazin-2-yl)-N″-(2-trifluormethoxy-phenylsulfonyl)-S-methyl-isothioharnstoff, 1,0 (0,02 Mol) Hydrazinhydrat und 100 ml Ethanol wird 60 Minuten bei 20 °C gerührt. Das hierbei kristallin angefallene Produkt wird durch Absaugen isoliert.

Man erhält 6,4 g (76 % der Theorie) N′-(4-Methyl-6-methoxy-s-triazin-2-yl-amino)-N″-(2-trifluormethoxyphenylsulfonyl)-guanidin vom Schmelzpunkt 245 °C.

Analog Beispiel 1 und 2 und gemäß den allgemeinen Angaben zur Herstellung der Verbindungen der Formel (I) können beispielsweise auch die in der nachstehenden Tabelle 2 aufgeführten Verbindungen der Formel (I) hergestellt werden:

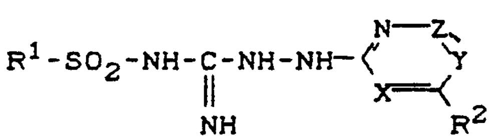

(I)

<u>Tabelle 2:</u> Herstellungsbeispiele für die Verbindungen der Formel (I)

| Bsp.-Nr | $R^1$ | $R^2$ | X | Y | Z | Schmelzpunkt (°C) |
|---|---|---|---|---|---|---|
| 3 | 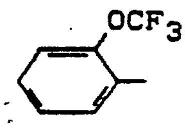 | $OCH_3$ | N | N | C-$OCH_3$ | 222 |
| 4 | 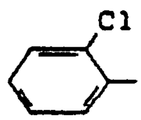 | $OCH_3$ | N | N | C-$OCH_3$ | 138 |
| 5 | 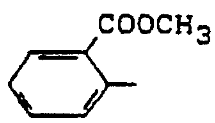 | $CH_3$ | N | N | C-$OCH_3$ | 230 |
| 6 | 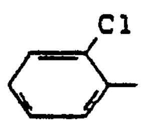 | $CH_3$ | N | N | C-$OCH_3$ | 208 |
| 7 | 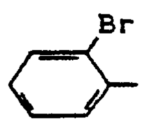 | $CH_3$ | N | N | C-$OCH_3$ | 195 |
| 8 | 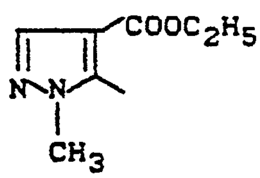 | $CH_3$ | N | CH | C-$OCH_3$ | 262 |
| 9 | 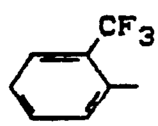 | $OCH_3$ | N | N | C-$OCH_3$ | 215 |
| 10 | 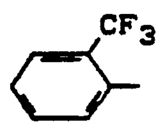 | $CH_3$ | N | N | C-$SCH_3$ | 202 |

Tabelle 2: - Fortsetzung

| Bsp.-Nr | $R^1$ | $R^2$ | X | Y | Z | Schmelzpunkt (°C) |
|---|---|---|---|---|---|---|
| 11 | 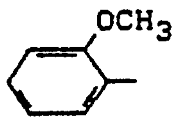 | $CH_3$ | N | N | $C-OCH_3$ | 235 |
| 12 | 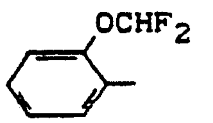 | $OCH_3$ | N | N | $C-OCH_3$ | 199 |
| 13 | 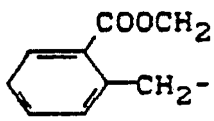 | $OCH_3$ | N | CH | $C-OCH_3$ | 195 |
| 14 | 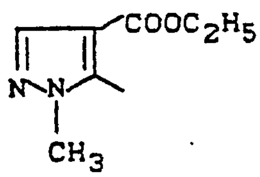 | $CH_3$ | N | CH | $C-CH_3$ | 270 |
| 15 | 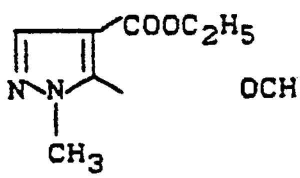 | $OCH_3$ | N | N | $C-OCH_3$ | 169 |
| 16 | 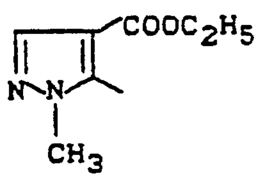 | $CH_3$ | N | N | $C-N(CH_3)_2$ | 193 |
| 17 | 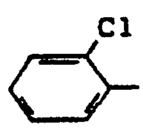 | $CH_3$ | N | N | $C-N(CH_3)_2$ | 245 |
| 18 | 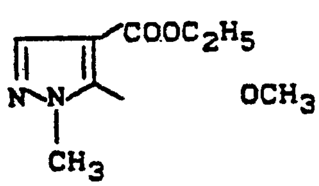 | $OCH_3$ | N | CH | $C-OCH_3$ | 162 |

Tabelle 2: - Fortsetzung

| Bsp.-Nr | $R^1$ | $R^2$ | X | Y | Z | Schmelzpunkt (°C) |
|---|---|---|---|---|---|---|
| 19 | 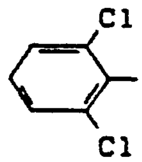 | $CH_3$ | N | N | $C\text{-}OCH_3$ | 243 |
| 20 | 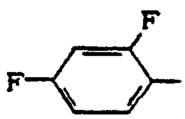 | $OCH_3$ | N | N | $C\text{-}OCH_3$ | 81 |
| 21 | 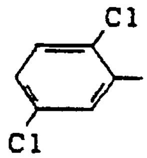 | $OCH_3$ | N | N | $C\text{-}OCH_3$ | 242 |
| 22 | 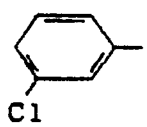 | $OCH_3$ | N | N | $C\text{-}OCH_3$ | 120 |
| 23 | 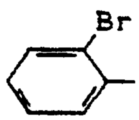 | $OCH_3$ | N | N | $C\text{-}OCH_3$ | 98 (Zers.) |
| 24 | 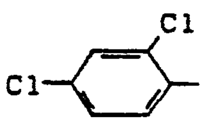 | $OCH_3$ | N | N | $C\text{-}OCH_3$ | 123 (Zers.) |
| 25 | 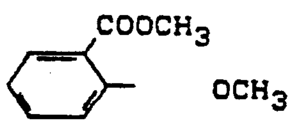 | $OCH_3$ | N | N | $C\text{-}OCH_3$ | 135 |
| 26 | 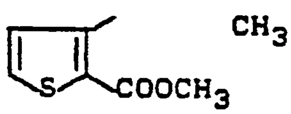 | $CH_3$ | N | N | $C\text{-}OCH_3$ | 198 |
| 27 | 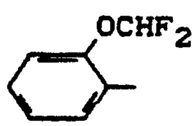 | $CH_3$ | N | N | $C\text{-}OCH_3$ | 240 |

Tabelle 2: - Fortsetzung

| Bsp.-Nr | $R^1$ | $R^2$ | X | Y | Z | Schmelzpunkt (°C) |
|---|---|---|---|---|---|---|
| 28 | 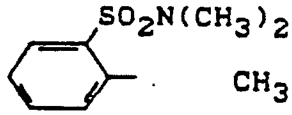 | $CH_3$ | N | N | C-$OCH_3$ | 215 |
| 29 | 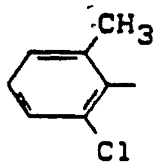 | $CH_3$ | N | N | C-$OCH_3$ | 188 |
| 30 | 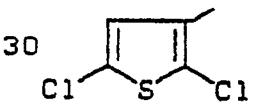 | $CH_3$ | N | N | C-$OCH_3$ | 210 |
| 31 | 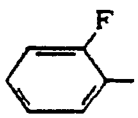 | $CH_3$ | N | N | C-$OCH_3$ | 191 |
| 32 | 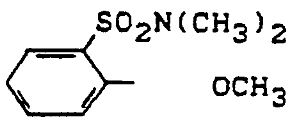 | $OCH_3$ | N | N | C-$OCH_3$ | 205 |
| 33 | 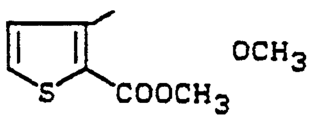 | $OCH_3$ | N | N | C-$OCH_3$ | 176 |
| 34 | 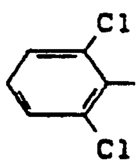 | $OCH_3$ | N | N | C-$OCH_3$ | >250 |
| 35 | 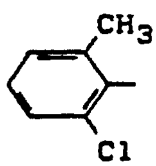 | $OCH_3$ | N | N | C-$OCH_3$ | 240 |
| 36 | 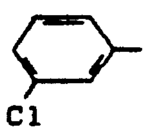 | $CH_3$ | N | N | C-$OCH_3$ | 209 |

Tabelle 2: - Fortsetzung

| Bsp.-Nr | $R^1$ | $R^2$ | X | Y | Z | Schmelzpunkt (°C) |
|---|---|---|---|---|---|---|
| 37 | 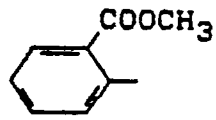 | $CH_3$ | N | N | $C-SCH_3$ | 200 |
| 38 | 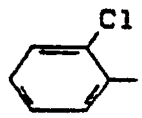 | $CH_3$ | N | N | $C-OC_2H_5$ | 153 |
| 39 | 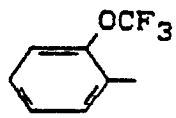 | $CH_3$ | N | N | $C-OC_2H_5$ | 240 |
| 40 | 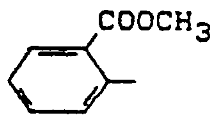 | $CH_3$ | N | N | $C-OC_2H_5$ | 166 |
| 41 | 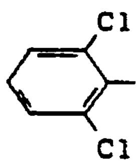 | $CH_3$ | N | N | $C-OC_2H_5$ | 213 |
| 42 | 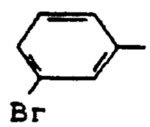 | $CH_3$ | N | N | $C-OCH_3$ | 195 |
| 43 | 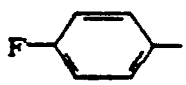 | $CH_3$ | N | N | $C-OCH_3$ | 220 |
| 44 | 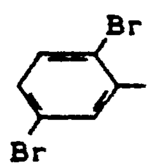 | $CH_3$ | N | N | $C-OCH_3$ | 248 |
| 45 | 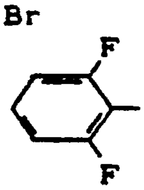 | $OCH_3$ | N | N | $C-OCH_3$ | 195 |

Tabelle 2: - Fortsetzung

| Bsp.-Nr | $R^1$ | $R^2$ | X | Y | Z | Schmelzpunkt (°C) |
|---|---|---|---|---|---|---|
| 46 | 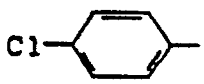 | $OCH_3$ | N | N | $C-OCH_3$ | 156 |
| 47 | 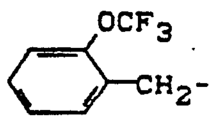 | $OCH_3$ | N | N | $C-OCH_3$ | 242 |
| 48 | 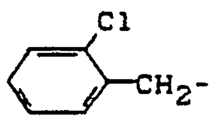 | $OCH_3$ | N | N | $C-OCH_3$ | 192 |
| 49 | 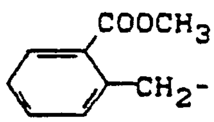 | $OCH_3$ | N | N | $C-OCH_3$ | 165 |
| 50 | 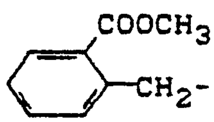 | $CH_3$ | N | N | $C-OCH_3$ | 227 |
| 51 | 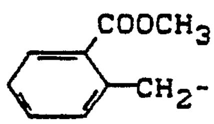 | $CH_3$ | N | N | $C-SCH_3$ | 235 |
| 52 | 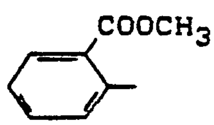 | $C_2H_5$ | N | N | $C-OCH_3$ | 190 |
| 53 | 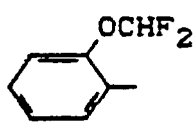 | $C_2H_5$ | N | N | $C-OCH_3$ | 227 |
| 54 | 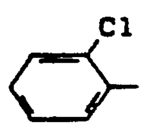 | $C_2H_5$ | N | N | $C-OCH_3$ | 127 |
| 55 | 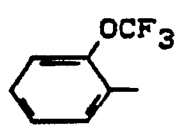 | $C_2H_5$ | N | N | $C-OCH_3$ | 238 |

Tabelle 2: - Fortsetzung

| Bsp.-Nr | $R^1$ | $R^2$ | X | Y | Z | Schmelzpunkt (°C) |
|---|---|---|---|---|---|---|
| 56 | 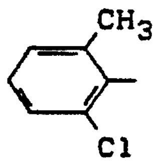 | $C_2H_5$ | N | N | $C-OCH_3$ | 204 |
| 57 | 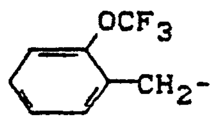 | $CH_3$ | N | N | $C-OCH_3$ | 221 |
| 58 | 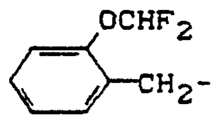 | $CH_3$ | N | N | $C-SCH_3$ | 207 |
| 59 | 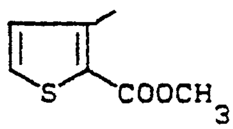 | $OCH_3$ | N | CH | $C-OCH_3$ | 179 |
| 60 | 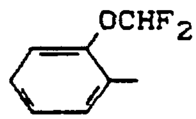 | $CH_3$ | N | CH | $C-CH_3$ | 185 |
| 61 | 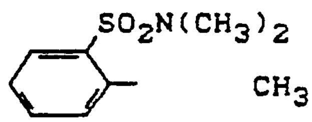 | $CH_3$ | N | CH | $C-CH_3$ | 214 |
| 62 | 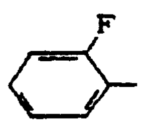 | $CH_3$ | N | CH | $C-CH_3$ | 202 |
| 63 | 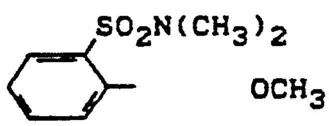 | $OCH_3$ | N | CH | $C-OCH_3$ | 194 |
| 64 | 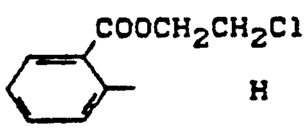 | H | N | CH | $C-CH_3$ | 145 |

Tabelle 2: - Fortsetzung

| Bsp.-Nr | $R^1$ | $R^2$ | X | Y | Z | Schmelzpunkt (°C) |
|---|---|---|---|---|---|---|
| 65 | 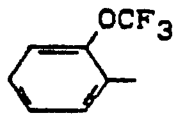 | H | N | CH | $C-CH_3$ | 132 |
| 66 | 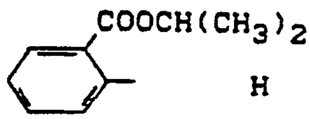 | H | N | CH | $C-CH_3$ | 150 |
| 67 | 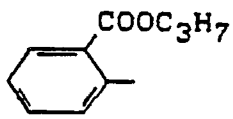 | H | N | CH | $C-CH_3$ | 128 |
| 68 | 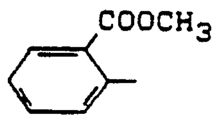 | H | N | CH | $C-CH_3$ | 184 |
| 69 | 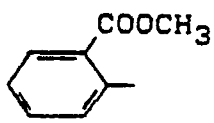 | $CH_3$ | N | CH | $C-CH_3$ | 172 |
| 70 | 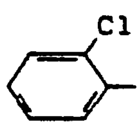 | $CH_3$ | N | CH | $C-OCH_3$ | 243 |
| 71 | 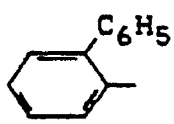 | $OCH_3$ | N | N | $C-OCH_3$ | 156 |
| 72 | 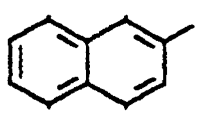 | $OCH_3$ | N | N | $C-OCH_3$ | 194 |
| 73 | 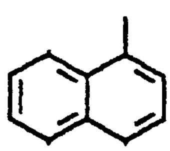 | $OCH_3$ | N | N | $C-OCH_3$ | 108 |

Tabelle 2: - Fortsetzung

| Bsp.-Nr | $R^1$ | $R^2$ | X | Y | Z | Schmelzpunkt (°C) |
|---|---|---|---|---|---|---|
| 74 | 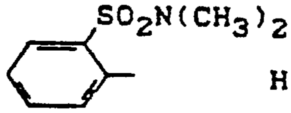 | H | N | CH | $C-CH_3$ | 148 |
| 75 | 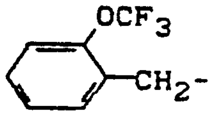 | $OCH_3$ | N | CH | $C-OCH_3$ | |
| 76 | 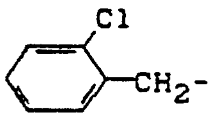 | $OCH_3$ | N | CH | $C-OCH_3$ | |
| 77 | 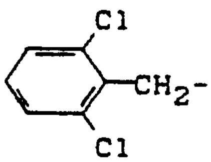 | $OCH_3$ | N | CH | $C-OCH_3$ | |
| 78 | 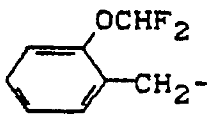 | $OCH_3$ | N | CH | $C-OCH_3$ | |
| 79 | 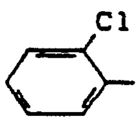 | $OCH_3$ | N | CH | $C-OCH_3$ | |
| 80 | 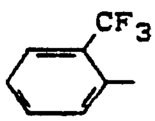 | $CH_3$ | N | CH | $C-CH_3$ | 189-193 |
| 81 | 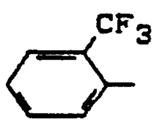 | $OCH_3$ | N | CH | $C-OCH_3$ | 162-166 |
| 82 | 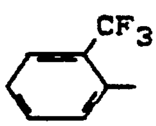 | H | N | CH | $C-CH_3$ | 208-211 |

<u>Tabelle 2:</u> - Fortsetzung

| Bsp.-Nr | $R^1$ | $R^2$ | X | Y | Z | Schmelzpunkt (°C) |
|---|---|---|---|---|---|---|
| 83 | 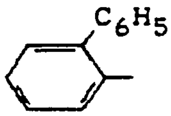 | $OCH_3$ | N | CH | $C-OCH_3$ | 234 |
| 84 | 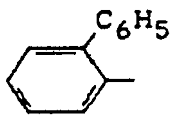 | $CH_3$ | N | N | $C-OCH_3$ | 219 |
| 85 | 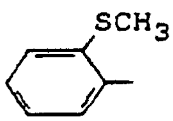 | $CH_3$ | N | CH | $C-CH_3$ | 120 |
| 86 | 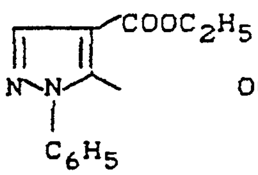 | $OCH_3$ | N | CH | $C-CH_3$ | 160 |
| 87 | 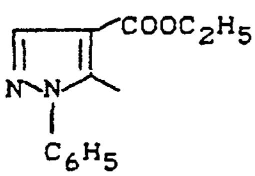 | $OCH_3$ | N | CH | $C-OCH_3$ | 148 |
| 88 | 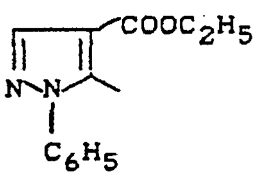 | $CH_3$ | N | CH | $C-CH_3$ | 240 |
| 89 | 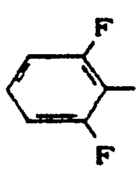 | $OCH_3$ | N | N | $C-CH_3$ | 230 (Zers.) |

Tabelle 2: - Fortsetzung

| Bsp.-Nr | $R^1$ | $R^2$ | X | Y | Z | Schmelzpunkt (°C) |
|---|---|---|---|---|---|---|
| 90 | 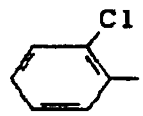 | $CH_3$ | N | N | $C-CH_3$ | 215 |
| 91 | 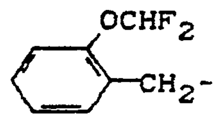 | $OCH_3$ | N | N | $C-CH_3$ | 203 |
| 92 | 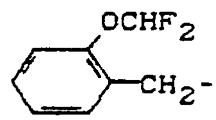 | $OCH_3$ | N | N | $C-C_2H_5$ | 179 |
| 93 | 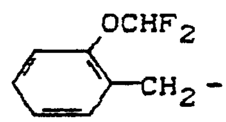 | $OC_2H_5$ | N | N | $C-CH_3$ | 168 |
| 94 | 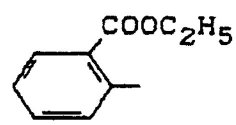 | $OC_2H_5$ | N | N | $C-CH_3$ | 178 (Zers.) |
| 95 | 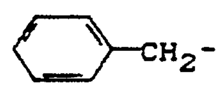 | $OCH_3$ | N | N | $C-CH_3$ | 237 (Zers.) |
| 96 | 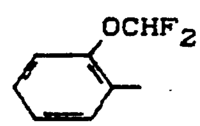 | $OCH_3$ | N | N | $C-CH_3$ | 248 (Zers.) |

<u>Tabelle 2:</u> - Fortsetzung

| Bsp.-Nr | $R^1$ | $R^2$ | X | Y | Z | Schmelzpunkt (°C) |
|---|---|---|---|---|---|---|
| 97 | 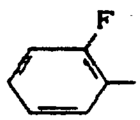 | $OCH_3$ | N | N | $C-OCH_3$ | 178 (Zers.) |
| 98 | 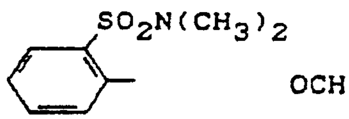 | $OCH_3$ | N | N | $C-CH_3$ | 235 (Zers.) |
| 99 | 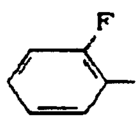 | $OCH_3$ | N | N | $C-C_2H_5$ | |
| 100 | 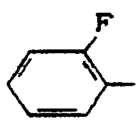 | $OC_2H_5$ | N | N | $C-CH_3$ | 250-253 (Zers.) |
| 101 | 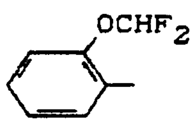 | $OC_2H_5$ | N | N | $C-CH_3$ | 251 (Zers.) |
| 102 | 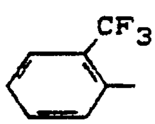 | $OC_2H_5$ | N | N | $C-CH_3$ | 220 (Zers.) |
| 103 | 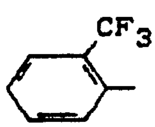 | $OCH_3$ | N | N | $C-CH_3$ | 224 |

Tabelle 2: - Fortsetzung

| Bsp.-Nr | $R^1$ | $R^2$ | X | Y | Z | Schmelzpunkt (°C) |
|---|---|---|---|---|---|---|
| 104 | 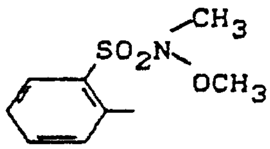 | $OCH_3$ | N | N | $C-CH_3$ | 213 (Zers.) |
| 105 | 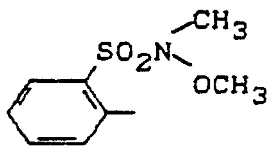 | $OCH_3$ | N | N | $C-OCH_3$ | 186 |
| 106 | 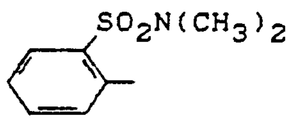 | $OCH_3$ | N | N | $C-C_2H_5$ | 190 (Zers.) |
| 107 | 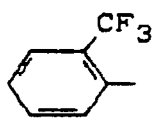 | $OCH_3$ | N | N | $C-C_2H_5$ | 222 |
| 108 | 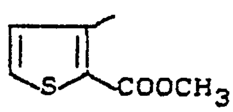 | $OCH_3$ | N | N | $C-C_2H_5$ | 100 (Zers.) |
| 109 | 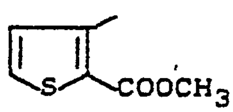 | $OC_2H_5$ | N | N | $C-CH_3$ | 110 (Zers.) |
| 110 | 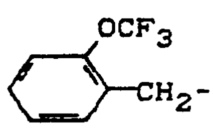 | $OCH_3$ | N | N | $C-C_2H_5$ | 260 |

Tabelle 2: - Fortsetzung

| Bsp.-Nr | $R^1$ | $R^2$ | X | Y | Z | Schmelzpunkt (°C) |
|---|---|---|---|---|---|---|
| 111 | 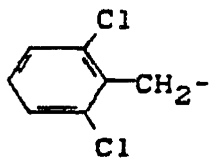 | $OCH_3$ | N | N | $C-CH_3$ | 258 (Zers.) |
| 112 | 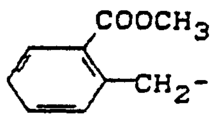 | $OCH_3$ | N | N | $C-C_2H_5$ | 178 (Zers.) |
| 113 | 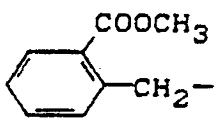 | $CH_3$ | N | N | $C-N(CH_3)_2$ | 206 (Zers.) |
| 114 | 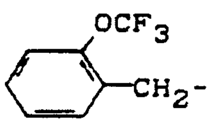 | $OC_2H_5$ | N | N | $C-CH_3$ | 282 (Zers.) |
| 115 | 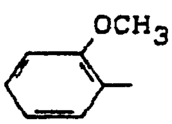 | $CH_3$ | N | CH | $C-CH_3$ | 201 |
| 116 | 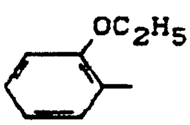 | $CH_3$ | N | CH | $C-CH_3$ | 127 |
| 117 | 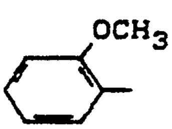 | $OCH_3$ | N | CH | $C-OCH_3$ | 160 |

Tabelle 2: - Fortsetzung

| Bsp.-Nr | $R^1$ | $R^2$ | X | Y | Z | Schmelzpunkt (°C) |
|---|---|---|---|---|---|---|
| 118 | 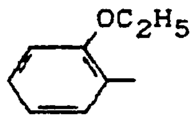 | $OCH_3$ | N | CH | $C-OCH_3$ | 212 |
| 119 | 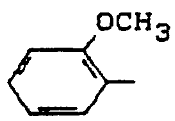 | $CH_3$ | N | CH | CH | 100 |
| 120 | 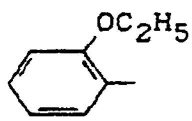 | $CH_3$ | N | CH | CH | 168 |
| 121 | 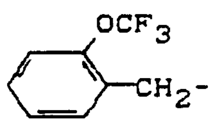 | $CH_3$ | N | CH | $C-CH_3$ | 236 |
| 122 | 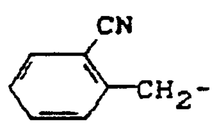 | $CH_3$ | N | CH | $C-CH_3$ | 243 |
| 123 | 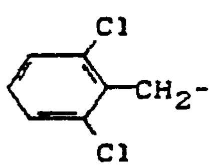 | $CH_3$ | N | CH | $C-CH_3$ | 235 |
| 124 | 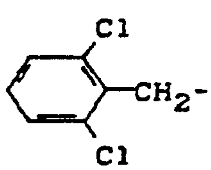 | $CH_3$ | N | CH | $C-CH_3$ | 214 |

Tabelle 2: - Fortsetzung

| Bsp.-Nr | $R^1$ | $R^2$ | X | Y | Z | Schmelzpunkt (°C) |
|---|---|---|---|---|---|---|
| 125 | 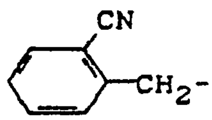 | $OCH_3$ | N | CH | $C-CH_3$ | 235 |
| 126 | 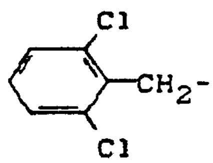 | $OCH_3$ | N | CH | $C-CH_3$ | 227 |
| 127 | 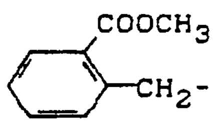 | $OCH_3$ | N | CH | $C-CH_3$ | 215 |
| 128 | 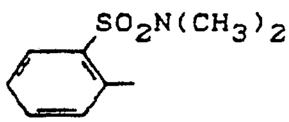 | $OCH_3$ | N | CH | $C-CH_3$ | 211 |
| 129 | 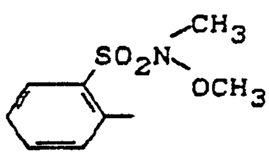 | $OCH_3$ | N | CH | $C-CH_3$ | 192 |
| 130 | 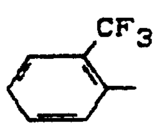 | $OCH_3$ | N | CH | $C-CH_3$ | 206 |
| 131 | 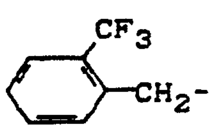 | $OCH_3$ | N | CH | $C-CH_3$ | 195 |

Tabelle 2: - Fortsetzung

| Bsp.-Nr | $R^1$ | $R^2$ | X | Y | Z | Schmelzpunkt (°C) |
|---|---|---|---|---|---|---|
| 132 | 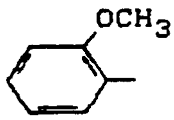 | $OCH_3$ | N | CH | $C-CH_3$ | 211 |
| 133 | 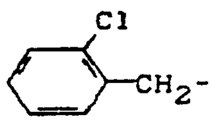 | $CH_3$ | N | CH | CH | 150 |
| 134 | 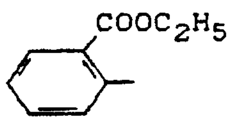 | $CH_3$ | N | CH | CH | 148 |
| 135 | 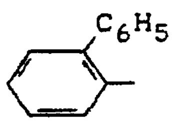 | $CH_3$ | N | CH | $C-CH_3$ | 136 |
| 136 | 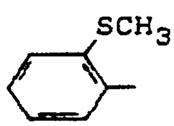 | $OCH_3$ | N | N | $C-OCH_3$ | 172 |
| 137 | 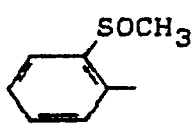 | $OCH_3$ | N | CH | $C-OCH_3$ | 222 |
| 138 | 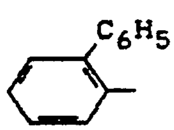 | $OCH_3$ | N | CH | $C-CH_3$ | 228 |

Tabelle 2: - Fortsetzung

| Bsp.-Nr | $R^1$ | $R^2$ | X | Y | Z | Schmelzpunkt (°C) |
|---|---|---|---|---|---|---|
| 139 | 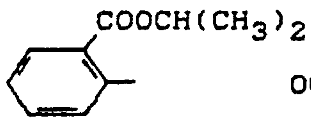 | $OCH_3$ | N | N | $C-OCH_3$ | 124 |
| 140 | 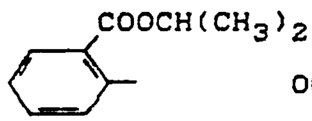 | $OCH_3$ | N | CH | $C-OCH_3$ | 149 |
| 141 | 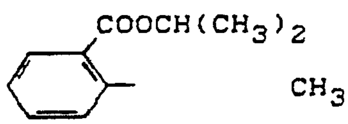 | $CH_3$ | N | CH | $C-CH_3$ | 158 |
| 142 | 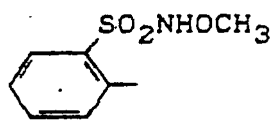 | $CH_3$ | N | CH | $C-CH_3$ | (amorph) |
| 143 | 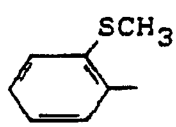 | $OCH_3$ | N | CH | $C-CH_3$ | (amorph) |

**Ansprüche**

1. Herbizide Mittel, gekennzeichnet durch einen Gehalt an mindestens einem sulfonylierten Aminoguanidin der allgemeinen Formel (I),

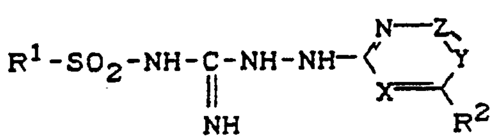

(I)

in welcher
$R^1$ für einen gegebenenfalls substituierten Rest aus der Reihe Aryl, Aralkyl und Heteroaryl steht,
$R^2$ für Wasserstoff, Halogen, Hydroxy, Amino oder einen gegebenenfalls substituierten Rest aus der Reihe Alkyl, Cycloalkyl, Alkoxy, Alkylthio, Alkylamino und Dialkylamino steht,
X für Stickstoff oder die Gruppierung CH steht,
Y für Stickstoff oder die Gruppierung C-$R^3$ steht, worin
$R^3$ für Wasserstoff, Halogen, Cyano, Alkyl, Formyl, Alkylcarbonyl oder Alkoxycarbonyl steht, und
Z für Stickstoff oder die Gruppierung C-$R^4$ steht, worin
$R^4$ für Wasserstoff, Halogen, Hydroxy, Amino oder einen gegebenenfalls substituierten Rest aus der Reihe Alkyl, Cycloalkyl, Alkoxy, Alkylthio, Alkylamino und Dialkylamino steht.

2. Herbizide Mittel, gekennzeichnet durch einen Gehalt an mindestens einem (zu den Verbindungen der

Formel (I) gemäß Anspruch 1 tautomeren) sulfonylierten Aminoguanidin der Formeln (IA) und/oder (IB),

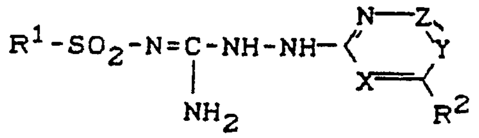

(IA)

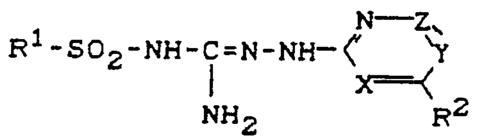

(IB)

worin
$R^1$, $R^2$, X, Y und Z die in Anspruch 1 angegebenen Bedeutungen haben.

3. Verfahren zur Bekämpfung von Unkraut, dadurch gekennzeichnet, daß man sulfonylierte Aminoguanidine der Formel (I) gemäß Anspruch 1 und/oder der Formeln (IA) und/oder (IB) gemäß Anspruch 2 auf die Unkräuter oder ihren Lebensraum einwirken läßt.

4. Verwendung von sulfonylierten Aminogruppen der Formel (I) gemäß Anspruch 1 und/oder der Formeln (IA) und/oder (IB) gemäß Anspruch 2 zur Bekämpfung von Unkraut.

5. Verfahren zur Herstellung von herbiziden Mitteln, dadurch gekennzeichnet, daß man sulfonylierte Aminoguanidine der Formel (I) gemäß Anspruch 1 bzw. der Formeln (IA) und/oder (IB) gemäß Anspruch 2 mit Streckmitteln und/oder oberflächenaktiven Stoffen vermischt.

Europäisches Patentamt

# EUROPÄISCHER RECHERCHENBERICHT

Nummer der Anmeldung

EP 90110761.5

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 5) |
|---|---|---|---|
| P,X | EP - A2 - 0 330 959 (BAYER AG) * Zusammenfassung; Beispiele (IV-2),(IV-3) und Tabelle 8, Verbindungen IVB-12 - IVB-22 ---- | 1-5 | A 01 N 47/44 |
| | | | RECHERCHIERTE SACHGEBIETE (Int Cl 5) |
| | | | A 01 N |

Der vorliegende Recherchenbericht wurde für alle Patentansprüche erstellt.

| Recherchenort | Abschlußdatum der Recherche | Prüfer |
|---|---|---|
| WIEN | 28-09-1990 | SCHNASS |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82